# EUROPEAN PATENT APPLICATION

(11) **EP 4 830 693 A1**
(43) Date of publication of application: **28.10.2026**
(21) Application number: 24907290.1
(22) Date of filing: 12.12.2024
(51) Int. Cl.: A61K 8/27, A61K 8/06, A61K 8/34, A61K 8/894, A61Q 17/04

(54) **AQUEOUS DISPERSION AND COSMETIC CONTAINING SAME**

(30) Priority: 22.12.2023 JP 2023216565
(71) Applicant: SHIN-ETSU CHEMICAL CO., LTD., Tokyo 100-0005 (JP)
(72) Inventor: MIYAUCHI, Masaru, Tokyo 100-0005 (JP); KONISHI, Masayuki, Tokyo 100-0005 (JP)
(74) Representative: Mewburn Ellis LLP
(86) International application number: PCT/JP2024/043946
(87) International publication number: WO 2025/134905

(57) **Abstract**

This aqueous dispersion has excellent dispersibility in aqueous media, exhibits excellent finish and coatability, and contains:
(a) 10 to 80 mass% hydrophobic zinc oxide fine particles that have been subjected to hydrophobization treatment with silicone and that have a number-average primary particle diameter of 8 to 200 nm as determined by image analysis of transmission electron microscope images;
(b) 1 to 50 mass% aqueous component having at least two alcoholic hydroxyl groups; and
(c) 1 to 20 mass% polyglycerin-modified silicone dissolved in the component (b).

## Description

### TECHNICAL FIELD

The present invention relates to an aqueous dispersion containing hydrophobized fine particles of zinc oxide, and to a cosmetic including the aqueous dispersion.

### BACKGROUND ART

In sunscreen cosmetics, inorganic powders, such as titanium dioxide and zinc oxide, are usually used in the form of fine particulate inorganic powder for the purposes of improving transparency and enhancing UV-shielding effects (Patent Document 1). These inorganic powders, however, have the propensity that the particles strongly interact with one another due to the large surface area and consequently are aggregated easily.

These fine particulate inorganic powders are often hydrophobized on the surface so that the powders can be blended into an oil phase of a cosmetic or their water resistance will be enhanced. The addition of such surface-hydrophobized powders is expected to provide UV-shielding effects that can be maintained even when wetted with water (Patent Documents 2 to 4). On the other hand, a fine particulate inorganic powder is sometimes hydrophilized on the surface with silica or the like when the powder is to be blended into an aqueous phase. Unfortunately, such hydrophilized powders have drawbacks in water resistance and the feeling on use. Studies on dispersing hydrophobized powders in water have led to the use of a polyether-modified silicone in an aqueous dispersion of hydrophobized fine particles of zinc oxide. However, such an aqueous dispersion of zinc oxide has drawbacks in water resistance and finish. Furthermore, sufficient comparative studies have not been made on aqueous zinc oxide dispersions (Patent Document 5).

In other studies, nonionic surfactants are added to dispersions, but this approach is unsuccessful in achieving sufficient dispersibility (Patent Document 6). For these reasons, there has been a demand for an aqueous dispersion that offers a good feeling on use when blended into a cosmetic.

### PRIOR ART DOCUMENTS

### PATENT DOCUMENTS

Patent Document 1: JP-A 2006-1886
Patent Document 2: JP-A 2014-201569
Patent Document 3: WO 2016/178380
Patent Document 4: JP-A 2020-002031
Patent Document 5: WO 2015/125622
Patent Document 6: JP-A H07-247119

### SUMMARY OF INVENTION

### TECHNICAL PROBLEM

The present invention has been made in consideration of the circumstances discussed above. It is therefore an object of the present invention to provide an aqueous dispersion that exhibits excellent dispersibility in an aqueous medium and offers an excellent finish and applicability. Another object of the present invention is to provide a cosmetic that includes the aqueous dispersion and excels in transparency and feeling on use (non-stickiness).

### SOLUTION TO PROBLEM

The present inventors carried out extensive studies directed to achieving the above objects. As a result, the present inventors have developed an aqueous dispersion that exhibits excellent dispersibility in an aqueous medium and offers an excellent finish and applicability. The present inventors have further found that when this dispersion is used as a dispersion in a cosmetic, the dispersion is easy to admit, and the cosmetic that is obtained has excellent transparency, feeling on use, and water resistance, thus completing the present invention.

Accordingly, the present invention provides an aqueous dispersion and a cosmetic containing the aqueous dispersion.
1. An aqueous dispersion including:
   (a) 10 to 80% by mass of hydrophobized fine particles of zinc oxide including zinc oxide particles hydrophobized with a silicone, the hydrophobized fine particles of zinc oxide having a number average primary particle diameter of 8 to 200 nm as determined by image analysis of a transmission electron photomicrograph;
   (b) 1 to 50% by mass of an aqueous component having at least two alcoholic hydroxyl groups; and
   (c) 1 to 20% by mass of a polyglycerin-modified silicone that dissolves in the component (b).
2. The aqueous dispersion according to 1, wherein the total content of the components (a), (b), and (c) in the aqueous dispersion is 90% by mass or more.
3. The aqueous dispersion according to 1 or 2, wherein the component (a) is hydrophobized fine particles of zinc oxide obtained by treating hydrous silica-coated zinc oxide particles with a silicone.
4. The aqueous dispersion according to any one of 1 to 3, wherein the silicone in the component (a) is triethoxycaprylylsilane.
5. The aqueous dispersion according to any one of 1 to 4, wherein the component (b) is an aqueous component having two alcoholic hydroxyl groups.
6. The aqueous dispersion according to any one of 1 to 5, wherein the component (c) is a component insoluble in water.
7. The aqueous dispersion according to any one of 1 to 6, wherein the component (c) is polyglyceryl-3 disiloxane dimethicone.
8. The aqueous dispersion according to any one of 1 to 7, wherein the mass ratio (c)/(b) of the content of the component (c) to the content of the component (b) is 0.2 to 0.9.
9. The aqueous dispersion according to 1, wherein the aqueous dispersion further includes (d) 8 to 82% by mass of water, and the total content of the components (a), (b), (c), and (d) in the aqueous dispersion is 90% by mass or more.
10. A cosmetic including the aqueous dispersion described in any one of 1 to 9.
11. The cosmetic according to 10, wherein the cosmetic is an emulsion including an aqueous phase including the aqueous dispersion, and the aqueous phase does not contain (e) hydrophobized fine particles of titanium dioxide that have been hydrophobized and have a number average primary particle diameter of 8 to 200 nm as determined by image analysis of a transmission electron photomicrograph.
12. The cosmetic according to 10, wherein the cosmetic is an emulsion including an aqueous phase including the aqueous dispersion, and an oil phase containing (e) hydrophobized fine particles of titanium dioxide that have been hydrophobized and have a number average primary particle diameter of 8 to 200 nm as determined by image analysis of a transmission electron photomicrograph.
13. The cosmetic according to 10, wherein the cosmetic is an emulsion including an aqueous phase including the aqueous dispersion, and the aqueous phase includes a dispersion of hydrophobized fine particles of titanium dioxide that includes:
   (e) hydrophobized fine particles of titanium dioxide that have been hydrophobized and have a number average primary particle diameter of 8 to 200 nm as determined by image analysis of a transmission electron photomicrograph;
   (b) an aqueous component having at least two alcoholic hydroxyl groups; and
   (c) a polyglycerin-modified silicone that dissolves in the component (b).

### ADVANTAGEOUS EFFECTS OF INVENTION

The aqueous dispersion according to the present invention exhibits excellent dispersibility in an aqueous medium and offers excellent applicability. When used as a dispersion in a cosmetic, the aqueous dispersion is easy to admit, and the cosmetic that is obtained has excellent transparency, feeling on use, and water resistance.

### DESCRIPTION OF EMBODIMENTS

Now the present invention is described in detail. Herein, the names of ingredients are sometimes expressed by the Japanese labeling name or the International Nomenclature of Cosmetic Ingredients (INCI). When the Japanese labeling name is identical with the INCI, sometimes either one is omitted.

### [Components (a)]

The component (a) in the present invention is hydrophobized fine particles of zinc oxide that include zinc oxide particles hydrophobized with a silicone and have a number average primary particle diameter of 8 to 200 nm as determined by image analysis of a transmission electron photomicrograph. The particles may be combined with other powder into a composite powder. Such components may be used singly, or two or more may be used in combination. Ultraviolet radiations are classified into long-wavelength ultraviolet radiations (UVA) with wavelengths of 320 to 400 nm, medium-wavelength ultraviolet radiations (UVB) with wavelengths of 290 to 320 nm, and short-wavelength ultraviolet radiations (UVC) with wavelengths of 290 nm and less. It is known that among the ultraviolet radiations, UVA and UVB reach the ground, and UVB causes erythema, etc. while UVA causes, for example, immediate darkening of the skin. In general, titanium dioxide has high UVB-shielding effects but does not shield UVA sufficiently. In contrast, zinc oxide is known to have high UVA-shielding effects. Thus, zinc oxide is an effective choice when a fine particulate inorganic powder is added to cosmetics to provide high UVA-shielding effects. The present invention has been successful in developing an aqueous dispersion that contains zinc oxide and exhibits excellent dispersibility in an aqueous medium.

In order to weaken aggregation or to reduce the activity of the powder, the zinc oxide particles may be surface-treated with, for example, silica, hydrous silica, or alumina, before the hydrophobic treatment. In particular, hydrous silica is preferable. That is, hydrous silica-coated zinc oxide particles are preferable.

Examples of the silicones used for the hydrophobic treatment include silanes or silylating agents, such as triethoxycaprylylsilane (AES-3083, manufactured by Shin-Etsu Chemical Co., Ltd.), dimethicone (KF-96 series, manufactured by Shin-Etsu Chemical Co., Ltd.), methylhydrogen polysiloxanes, such as hydrogen dimethicone (for example, KF-99P and KF-9901, manufactured by Shin-Etsu Chemical Co., Ltd.), and silicone-branched silicones, such as triethoxysilylethyl polydimethylsiloxyethyl dimethicone and triethoxysilylethyl polydimethylsiloxyethylhexyl dimethicone (for example, KF-9908 and KF-9909, manufactured by Shin-Etsu Chemical Co., Ltd.). In particular, the component including at least one selected from triethoxycaprylylsilane, hydrogen dimethicone, and dimethicone is preferable because such an agent can achieve satisfactory hydrophobic treatment and ensures good dispersibility with the component (c). In particular, triethoxycaprylylsilane and dimethicone are preferable. Triethoxycaprylylsilane is more preferable from the point of view of compatibility. The hydrophobic treatment method is not particularly limited and may be any well-known method, with examples including wet treatment methods, dry treatment methods, and gas phase methods.

Such surface-treated fine particulate inorganic powders may be commercially available products. For example, such powders are available in the market under the following product names: MZ-306X, 506X, MZY-203S, 210M3S, TMZ-HA1, MZX-203OTS, 304OTS, 508OTS, MZN-A1, ZEC-1, MZX-300M, and 505HPS (manufactured by TAYCA Co., Ltd.), FINEX-50W-LP2, 52W-LP2, 30W-LP2, 33W-LP2, 50W-LP2, 25-LPT, 30S-LP2, and 50S-LPT (manufactured by SAKAI CHEMICAL INDUSTRY CO., LTD.), and ZNO XZ-11S3L, ZnO-660SS-11S5, ZNO-660-ASGP7, MZO-35-11S5, MZO-35-I3, MZO-35-NOE7, ZnO-750-11SP, ZNO-750-ASG5, ZnO-750-ASGP6, ZnO-750-NJE7, ZNO FSF-11S4, A120-ZNO-11S3, and ZnO-USP1-I2 (manufactured by KOBO).

The number average primary particle diameter of the component (a) determined by image analysis of a transmission electron photomicrograph is 8 to 200 nm, preferably 10 to 150 nm, and more preferably 20 to 100 nm. If the number average primary particle diameter is more than 200 nm, the UV protection function is lowered and the powder applied leaves a white cast on the skin. If the particle diameter is less than 8 nm, dryness is increased and may adversely affect the feeling on use. The number average primary particle diameter of the component (a) in the present invention is the average diameter of 200 particles measured by image analysis based on a transmission electron photomicrograph. When the powder is not spherical, the number average primary particle diameter is the average of the minor axe and major axe of the particles.

The content of the component (a) in the aqueous dispersion is 10 to 80% by mass. From the point of view of usability, the content is preferably 20 to 75% by mass, more preferably 25% by mass or more, still more preferably 30% by mass or more, and particularly preferably 50% by mass or more. On the other hand, the content is preferably 70% by mass or less, and more preferably 65% by mass or less. If the content is less than 10% by mass, sufficient UV-shielding effects cannot be obtained. If the content is more than 80% by mass, there is a risk that spreadability on use is deteriorated or the dispersion loses age stability and experiences a viscosity buildup.

### [Components (b)]

The component (b) in the present invention is an aqueous component having at least two alcoholic hydroxyl groups, specifically, a component that dissolves in water at 25°C. Such components may be used singly, or two or more may be used in combination. Alcohols having one hydroxyl group, such as ethanol and propanol, do not provide sufficient dispersibility in water. Specific examples of the components (b) include sugar alcohols, such as Sorbitol (INCI), Maltose (INCI), Xylitol (INCI), Glucose (INCI), Glyceryl Glucoside (INCI), sodium chondroitin sulfate (labeling name, INCI: Sodium Chondroitin Sulfate), Methyl Gluceth-10 (INCI), Methyl Gluceth-20 (INCI), hyaluronic acid, phosphatidylglycerol, and phosphatidylinositol; glycols, such as BG (labeling name, INCI: Butylene Glycol), PG (labeling name, INCI: Propylene Glycol), DPG (labeling name, INCI: Dipropylene Glycol), Pentylene Glycol (INCI), 1,10-Decanediol (INCI), Octanediol (INCI), and 1,2-Hexanediol (INCI); and polyhydric alcohols, such as Erythritol (INCI), Glycerin (INCI), Diglycerin (INCI), and polyethylene glycol. Of these, such glycols as BG (butylene glycol) and DPG (dipropylene glycol), and such polyhydric alcohols as glycerin are preferable because they dissolve in water in any proportion and also in view of the feeling on use and the versatility as cosmetic ingredients. In particular, aqueous components having two alcoholic hydroxyl groups in the molecule, and glycols are more preferable.

The content of the component (b) in the aqueous dispersion is 1 to 50% by mass, preferably 5 to 25% by mass, and, from the point of view of water resistance, more preferably 7 to 15% by mass. From the point of view of stability over time, the content is still more preferably 15 to 25% by mass, and particularly preferably 15 to 20% by mass. If the content is less than 1% by mass, the dispersion becomes less stable. If the content is more than 50% by mass, a cosmetic containing such an aqueous dispersion gives a sticky feeling on use, and the component (b) comes to prevent the component (c) described later from orienting toward the component (a), which may lead to a high viscosity or deterioration in dispersibility in an aqueous medium. The component (b) helps the component (c) uniformly orient on the surface of the component (a).

### [Components (c)]

The component (c) in the present invention is a polyglycerin-modified silicone that dissolves in the component (b). Such components may be used singly, or two or more may be used in combination. By the use of the polyglycerin-modified silicone that dissolves in the component (b), the polyglycerin-modified silicone can be blended in water and can function as a dispersant for the hydrophobized fine particles of zinc oxide in water. Regarding the phrase that the component (c) "dissolves in the component (b)", the component (c) "dissolves" when a mixture of 20% by mass of the component (c) with the component (b), after being allowed to stand at 25°C for 1 hour, is transparent to semi-transparent without any boundary, whereas the component (c) does "not dissolve" when the mixture becomes white turbid or separates into two layers. Being "transparent to semi-transparent" means that the total light transmission through a 1 cm thick cell filled with the mixture liquid is 50% or more as measured in accordance with the method described in JIS K7361-1: 1997. Those polyglycerin-modified silicones that do not dissolve in the component (b) do not belong to the components (c) in the present invention.

The chemical structure of the polyglycerin-modified silicone may be such that the silicone chain as the backbone is modified with a block or a graft of polyglycerin. In order to maintain uniform dispersibility of the component (a) in a cosmetic, it is preferable to use a branch-modified silicone. The silicone backbone may have a branched chain, such as a silicone chain. Specific examples include polyglyceryl-3 disiloxane dimethicone (labeling name, INCI: Polyglyceryl-3 Disiloxane Dimethicone). From the point of view of water resistance, the component (c) is preferably one that dissolves at 25°C when mixed in butylene glycol (BG) at a concentration of 20% by mass, and does not dissolve under the above condition when mixed in water at a concentration of 20% by mass. The polyglycerin-modified silicone in the present invention may be co-modified with hydrophilic groups other than polyglycerin groups, but 50% by mass or more of the hydrophilic groups are polyglycerin groups. From the point of view of water resistance, a silicone exclusively modified with polyglycerin groups is preferable.

The polyglycerin-modified silicone used in the present invention preferably has an HLB (hydrophile-lipophile balance) value of 3 or more and less than 17, more preferably 5 or more and less than 15, and still more preferably 8 or more and less than 12. When the value is 3 or more, dispersibility is further enhanced. When the value is less than 17, water resistance is further enhanced. In the present invention, the HLB is calculated from "(sum of formula weights of hydrophilic group moieties/total molecular weight) × 20".

The content of the component (c) in the aqueous dispersion is 1 to 20% by mass, preferably 3 to 15% by mass, more preferably 4 to 10% by mass, and still more preferably 5 to 8% by mass. If the content is less than 1% by mass, the dispersion may become less stable. If the content is more than 20% by mass, molecules of the component (c) may be oriented on the component (a) that already has molecules of the component (c) oriented thereon, or molecules of the component (c) that have been oriented on the component (a) may be easily detached by other molecules of the component (c) not oriented on the component (a). Possible consequences are an increased viscosity of the dispersion and adverse effects on dispersibility in an aqueous medium.

Furthermore, the mass ratio (c)/(b) of the content of the component (c) to the content of the component (b) is preferably 0.2 to 0.9. From the points of view of dispersibility and water resistance, the ratio is more preferably 0.2 to 0.5, and still more preferably 0.2 to 0.35. By controlling the ratio to 0.2 or more, the component (c) can easily orient on the component (a) and the dispersion is prevented from undergoing a viscosity buildup and becomes more stable. On the other hand, controlling the ratio to 0.9 or less ensures that the component (c) is fully dispersed in the dispersion medium and can easily orient on the component (a), and the dispersion is prevented from undergoing a viscosity buildup and becomes more stable.

The total content of the components (a), (b), and (c) in the aqueous dispersion is preferably 90% by mass or more, more preferably 95% by mass or more, and still more preferably 99% by mass or more. When the component (d) described below is not added, the upper limit may be 100% by mass. By controlling the total content to 90% by mass or more, the influence of components other than the components (a), (b), and (c) can be reduced, and the dispersion thus prepared attains higher stability and a better feeling on use.

### [Components (d)]

Water may be added to the aqueous dispersion of the present invention. Examples of water include ion exchanged water, distilled water, deionized water, purified water defined in the Japanese Pharmacopoeia, spring water, deep ocean water, and plant extract water.

When the component (d) is added, the content thereof in the aqueous dispersion is preferably 8 to 82% by mass, more preferably 18 to 72% by mass, and still more preferably 28 to 40% by mass. Adding the component (d) can lower the viscosity of the aqueous dispersion. From the point of view of the stability of the aqueous dispersion over time, the content is preferably 82% by mass or less.

When the component (d) is added, the total content of the components (a), (b), (c), and (d) in the aqueous dispersion is preferably 90% by mass or more, more preferably 95 to 100% by mass, and still more preferably 99 to 100% by mass. By controlling the total content of the components (a), (b), (c), and (d) to 90% by mass or more, the influence of components other than the components (a), (b), (c), and (d) can be reduced, and the dispersion thus prepared attains higher stability and a better feeling on use.

An antifoaming agent may be added to the aqueous dispersion to enhance handling properties at the time of preparation, filling, or the like. Examples of the antifoaming agents include Simethicone (INCI), Dimethicone (INCI), salts, such as sodium chloride (labeling name, INCI: Sodium Chloride), and polyether-modified silicones. Oils, such as dimethicone, may be used as previously emulsified emulsion types, and compounds, such as simethicone, may be used as self-emulsifying types previously mixed with silicone surfactants or the like. Among those described above, in particular, simethicone and sodium chloride are preferable in terms of defoaming properties and antifoaming properties. Simethicone is preferable in terms of the affinity for cosmetic ingredients. When the antifoaming agent is added, the amount thereof in the aqueous dispersion is preferably 0.0001 to 1% by mass, more preferably 0.0025 to 0.6% by mass, and still more preferably 0.005 to 0.01% by mass.

### [Aqueous dispersions]

The aqueous dispersion of the present invention can be easily blended into an aqueous medium. The term "dispersion" in the "aqueous dispersion" means that the mixture having a formulation including 8 to 99% by mass of water shows temporary sedimentation when tested by the dispersibility test described later in Examples, but becomes uniformly dispersed upon continued stirring.

### [Properties of the aqueous dispersions]

The dispersion of the present invention is preferably a liquid having a viscosity of 10 mPa·s or more and less than 200,000 mPa·s, more preferably 10 mPa·s or more and less than 10,000 mPa·s, and still more preferably 10 mPa·s or more and less than 5,000 mPa·s. By controlling the viscosity to 10 mPa·s or more, the stability of the dispersion is further enhanced. On the other hand, a viscosity of less than 200,000 mPa·s is preferable from the point of view of handling properties. The viscosity is a value measured at 25°C using a Brookfield viscometer (TVB-10, manufactured by Toki Sangyo Co., Ltd.) in accordance with the method described in JIS K7117-1: 1999.

When the dispersion is a paste, the hardness is not particularly limited. When the production is made with a three-roll mill, the hardness is preferably 100 or less to facilitate the processing. The hardness is more preferably less than 80, and still more preferably less than 50 to facilitate blending in cosmetics. The lower limit is not particularly limited, but the concentration variation in the dispersion tends to be small when the hardness is 5 or more. The hardness is a value measured with a rheometer, for example, Rheometer RT-2002D·D (manufactured by RHEOTECH, probe: 5 mmφ, penetration depth: 10 mm, sample stage rising speed: 5 cm/min, temperature: 25°C, range: 200).

### [Methods for producing the aqueous dispersion]

The aqueous dispersion of the present invention may be prepared using any known method and apparatus without limitation. For example, use may be made of any agitators, mills, mixers, media agitating mills, planetary centrifugal agitators, and dispersing machines, such as Henschel mixers, ball mills, kneaders, planetary mixers, ribbon blenders, dispersing mixers, homomixers, jet mills, roll mills, bead mills, and high-pressure dispersers. From the point of view of mixing efficiency, it is preferable that the components be dispersed using a bead mill or a high-pressure disperser.

### [Cosmetics]

The aqueous dispersion of the present invention may be used in a variety of applications and, in particular, is usable as an ingredient for all cosmetics that are externally applied to the skin and hair.

The amount of the aqueous dispersion of the present invention in the cosmetic is preferably 0.5 to 60% by mass, more preferably 1.0 to 50% by mass, and still more preferably 5.0 to 40% by mass. By controlling the amount to 0.5% by mass or more, sufficient UV protection effects can be expected. By controlling the amount to 60% by mass or less, the feeling on use is further enhanced.

The type of the cosmetic containing the aqueous dispersion of the present invention is not particularly limited. For example, aqueous cosmetics (cosmetics in which the bases are aqueous components (water, water-soluble components, and water-dispersible components)) and emulsions are preferable. The emulsions may be any of, for example, W/O (water-in-oil) emulsion cosmetics, O/W (oil-in-water) emulsion cosmetics, and multiphase emulsions, such as W/O/W and O/W/O. W/O emulsion cosmetics are preferable in terms of water resistance. When, in particular, the dispersion is used in an O/W emulsion cosmetic, the dispersion is easy to admit and the cosmetic that is obtained is stable and has excellent transparency, feeling on use, and water resistance.

### [Components (e)]

The cosmetic of the present invention may include (e) hydrophobized fine particles of titanium dioxide that have been hydrophobized and have a number average primary particle diameter of 8 to 200 nm as determined by image analysis of a transmission electron photomicrograph. This component is expected to shield short-wavelength light, and the cosmetic that is prepared is expected to have higher UV-shielding effects. The hydrophobic treatment is not particularly limited. Those treatment agents used in the silicone treatment for zinc oxide described hereinabove, and any known treatment agents generally used in cosmetics may be used without limitation. Examples include waxes, paraffins, organic fluorine compounds, such as perfluoroalkyl phosphate salts, surfactants, amino acids, such as N-acyl glutamic acid, and metal soaps, such as aluminum stearate and magnesium myristate. In particular, one or more selected from stearic acid, isostearic acid, and triethoxycaprylylsilane may be used. The hydrophobic treatment method is not particularly limited and may be any well-known method, with examples including wet treatment methods, dry treatment methods, and gas phase methods.

In order to weaken aggregation or to reduce the activity of the powder, the fine particles of titanium dioxide may be surface-treated with, for example, silica, hydrous silica, alumina, or aluminum hydroxide, before the hydrophobic treatment. The use of alumina or aluminum hydroxide as the surface treatment agent is not preferable in the case where there is a concern that such a surface treatment agent may inhibit the swelling of a water-soluble polymer compound added together with the powder in the cosmetic, or may cause a decrease in water resistance. Hydrous silica-coated titanium dioxide particles that are surface-treated with hydrous silica are preferable because hydrous silica suppresses the activity of the powder and is unlikely to inhibit the swelling of a water-soluble polymer. The term "coated" means that the titanium dioxide particles are coated partially or completely.

Such surface-treated fine particles of titanium dioxide may be commercially available products. For example, such fine particles are available in the market under the following product names: MT-01, 02, 050OTS, 100Z, 100TV, 100SAS, 150EX, 200ST, 500SAM, 505SAS, 700Z, 700BS, N1, and 500CST (manufactured by TAYCA Co., Ltd.), ST-455, 455WS, 457ECS, 457SA, 495M, and 455FA (manufactured by Titan Kogyo, Ltd.), and STR-100A-LP, 100C-LP, 100W-LP, 100C-LF, and 40-LP (manufactured by Sakai Chemical Industry Co., Ltd.). Examples of the hydrophobized fine particles of titanium dioxide obtained by hydrophobizing hydrous silica-coated titanium dioxide particles include STR-100W-LP.

The number average primary particle diameter of the component (e) determined by image analysis of a transmission electron photomicrograph is 8 to 200 nm, and preferably 10 to 150 nm. If the number average primary particle diameter is more than 200 nm, the UV protection function is lowered and the powder applied may leave a white cast on the skin. If the particle diameter is less than 8 nm, dryness is increased and may adversely affect the feeling on use. The number average primary particle diameter of the component (e) in the present invention is the average diameter of 200 particles measured by image analysis based on a transmission electron photomicrograph. When the powder is not spherical, the number average primary particle diameter is the average of the minor axe of the particles. For example, the fine particles of titanium dioxide may be of spindle, needle, bunch, strip, substantial sphere, or rod shape.

When the component (e) is added, the content thereof in the cosmetic is preferably 1 to 30% by mass, and more preferably 5 to 15% by mass. When the component (e) is added, it is preferable that the component (e) be dispersed in an oil beforehand.

When an emulsion is formed using the aqueous dispersion of the present invention, it is preferable that the aqueous dispersion be added into the aqueous phase. When the aqueous dispersion of the present invention is used to form an aqueous cosmetic or an emulsion, in particular, a water-in-oil (W/O) cosmetic, it is preferable from the points of view of the transparency and the feeling on use of the cosmetic that the component (e) be not added into the aqueous phase, and it is more preferable that the component (e) be added into the oil phase. In this manner, deterioration in stability and the feeling on use stemming from hetero-aggregation of zinc oxide (a) and titanium dioxide (e) in the aqueous dispersion can be prevented.

When the component (e) is added, for example, the components (b), (c), and (e) may be formed into an aqueous dispersion beforehand, or the components (b), (c), (d), and (e) may be formed into an aqueous dispersion beforehand in the same manner as the aqueous dispersion of the present invention. Specifically, the component (e) may be added in the form of a dispersion of hydrophobized fine particles of titanium dioxide that includes:
(e) hydrophobized fine particles of titanium dioxide that have been hydrophobized and have a number average primary particle diameter of 8 to 200 nm as determined by image analysis of a transmission electron photomicrograph;
(b) an aqueous component having at least two alcoholic hydroxyl groups; and
(c) a polyglycerin-modified silicone that dissolves in the component (b). In this manner, the UV-shielding effects are further enhanced while effectively ensuring the transparency, the feeling on use, and the water resistance of the cosmetic. The dispersion of hydrophobized fine particles of titanium dioxide is preferably an aqueous dispersion.

The content of the component (e) in the dispersion is preferably 10 to 80% by mass of the dispersion, and more preferably 10 to 70% by mass. From the point of view of usability, the content is still more preferably 10 to 65% by mass, particularly preferably 15 to 60% by mass, and most preferably 20 to 55% by mass. From the point of view of UV-shielding effects, the content is preferably 10% by mass or more. If the content is more than 80% by mass, there is a risk that spreadability on use is deteriorated or the dispersion loses age stability and experiences a viscosity buildup. Preferred examples and preferred amounts of the components (b), (c), and (d) are the same as those described with respect to the aqueous dispersion hereinabove. When, in particular, the dispersion containing the component (e) is an aqueous dispersion, the content of the component (b) is preferably in the range of 1.0 to 30% by mass, and the content of the component (c) is preferably in the range of 1.0 to 20% by mass.

The form of the cosmetic of the present invention may be selected from various forms including liquid, milky lotion, cream, solid, paste, gel, powder, pressed, multilayer, mousse, spray, stick, and pencil. The multilayer form refers to a cosmetic that separates into two or more layers upon static holding. Such a cosmetic is placed in a container containing stainless steel balls or the like, and is used after shaking the container. The cosmetic of such a form is called the shaking type or the like. Such a cosmetic easily reaches a stabilized state and gives a pleasant feeling on use, but the need of shaking can be troublesome. The aqueous dispersion of the present invention has good dispersibility and can be easily re-dispersed even in such a form of cosmetic. The cosmetic of the present invention has good stability and can be used without separating into multiple layers. The spray form refers to a spraying cosmetic that is placed in a dispenser container, an aerosol container, or the like and is used by being sprayed through a nozzle. The cosmetic placed in a dispenser container is sprayed as mist through a nozzle on the dispenser. The cosmetic is placed in the aerosol container together with a spraying agent. The spraying agent is not particularly limited and may be selected from, for example, various liquefied petroleum gases (LPG), dimethyl ether, nitrogen gas, and carbon dioxide gas. The spraying agents may be used singly, or two or more may be used in appropriate combination. By virtue of its high dispersibility, the aqueous dispersion of the present invention can be used even in this form of cosmetic. Properties, such as pH and viscosity, of the cosmetic are selected appropriately in accordance with the form.

The aqueous dispersion of the present invention is applicable to a variety of cosmetics, particularly preferably to cosmetics externally applied to the skin, such as skin care cosmetics, make-up cosmetics, antiperspirant cosmetics, and UV-protecting cosmetics, and cosmetics externally applied to the hair, such as hair care cosmetics. Exemplary skin care cosmetics include face lotions, milky lotions, creams, cleansing agents, packs, oil liquids, massage creams, cosmetic liquids, cosmetic oils, detergents, deodorants, hand creams, lip creams, and anti-wrinkle agents. Exemplary make-up cosmetics include make-up foundations, concealers, cosmetic powders, powder foundations, eye colors, eye shadows, mascaras, eye liners, eyebrow cosmetics, and lip cosmetics. Exemplary antiperspirant cosmetics include antiperspirant cosmetics of roll-on type, cream type, solution type, and stick type. Exemplary UV-protecting cosmetics include sunscreen oils, sunscreen milky lotions, and sunscreen creams. Exemplary hair care cosmetics include shampoos, conditioners, treatments, and setting agents. Among these, the UV-protecting cosmetics are preferable. Properties, such as pH and viscosity, of the cosmetic are selected appropriately in accordance with the formulation.

The cosmetic of the present invention may contain various components that are commonly used in cosmetics as long as the advantageous effects of the present invention are not impaired. Suitable components include, for example, (1) oils, (2) aqueous components other than the components (b) and (d), (3) surfactants other than the components (c), (4) powders other than the components (a) and (e), (5) compositions consisting of a crosslinked organopolysiloxane and an oil that is liquid at room temperature, (6) film-forming agents, and (7) other additives. These may be used singly, or two or more may be used in appropriate combination. The components used in the dispersion described hereinabove may be added. However, the marked effects aimed at in the present invention cannot be obtained even if the components (a) to (c) in the present invention are added unless the dispersion of the present invention is added. The amounts of the components (a) to (c) not forming the dispersion are not particularly limited as long as the advantageous effects of the present invention are not impaired. When these components are added to the cosmetic separately from the dispersion, the amounts excluding the amounts in the dispersion are, for example, as follows. The amount of the component (a) is preferably 0.0 to 20% by mass, and more preferably 0.0 to 10% by mass of the cosmetic. The amount of the component (b) is preferably 0.0 to 70% by mass, more preferably 0.0 to 50% by mass, and still more preferably 0.0 to 25% by mass of the cosmetic. The amount of the component (c) is preferably 0.0 to 3.0% by mass, and more preferably 0.0 to 1.0% by mass of the cosmetic. The dispersion of the present invention is highly stable and can be added to a cosmetic without inhibiting the performance of an emulsifier or a dispersant in the cosmetic, thus allowing the amount of the component (c) other than in the dispersion to be reduced.

### (1) Oils

The oils may be volatile or nonvolatile and may be solid, semisolid, or liquid at room temperature (25°C). Examples include silicone oils, solid oily components, natural animal and plant oils, semi-synthetic oils, hydrocarbon oils, higher alcohols, fatty acids, ester oils, fluorinated oils, and UV absorbers.

### • Silicone oils

Examples of the silicone oils include Dimethicone (INCI), Trisiloxane (INCI), alkyl-modified silicones, such as Methyl Trimethicone (INCI), Ethyl Trisiloxane (INCI), Ethyl Methicone (INCI), and Hexyl Dimethicone (INCI), long-chain alkyl-modified silicones, such as Caprylyl Methicone (INCI), low- to high-viscosity linear or branched organopolysiloxanes, such as Phenyl Trimethicone (INCI), Diphenyl Dimethicone (INCI), Diphenylsiloxyphenyl Trimethicone (INCI), Tetraphenyl Dimethyldisiloxane (INCI), and methyl hydrogen polysiloxane, cyclic organopolysiloxanes, such as Cyclotetrasiloxane (INCI), Cyclopentasiloxane (INCI), and Cyclohexasiloxane (INCI), amino-modified organopolysiloxanes, such as Amodimethicone (INCI) and Aminopropyl Dimethicone (INCI), pyrrolidone-modified organopolysiloxanes, such as PCA Dimethicone (INCI), antifoaming agents, such as Simethicone (INCI), pyrrolidone carboxylic acid-modified organopolysiloxanes, silicone rubbers, such as gum-like dimethylpolysiloxanes having a high degree of polymerization, gum-like amino-modified organopolysiloxanes, and gum-like dimethylsiloxane-methylphenylsiloxane copolymers, as well as low-viscosity organopolysiloxane solutions of silicone gums and rubbers, amino acid-modified silicones, fluorine-modified silicones, silicone resins, and silicone resin solutions.

Commercially available examples of the silicone oils include KF-96L-1cs, KF-96L-1.5cs, KF-96L-2cs, KF-96A-6cs, KF-4422, KF-4418, KF-54, KF-54HV, KF-56A, and KF-995 manufactured by Shin-Etsu Chemical Co., Ltd.

### • Solid oily components

When it is desired that the cosmetic of the present invention be solidified, an oily component that is solid at 25°C is preferably blended. Examples of the oily components that are solid at 25°C include waxes, hydrocarbons, esters, higher alcohols, and higher fatty acids having a melting point of preferably at least 40°C, more preferably 60 to 110°C. The oily component is not particularly limited as long as it is commonly blended in cosmetics. Specific examples include plant waxes, such as carnauba wax (INCI: Copernicia Cerifera (Carnauba) Wax), sugar cane wax, candelilla wax (INCI: Euphorbia Cerifera (Candelilla) Wax), purified candelilla wax, rice wax, Japan wax, jojoba wax, kapok wax, rice bran wax, myrica cerifera fruit wax, shea butter, cacao butter, Japan wax (INCI: Rhus Succedanea Fruit Wax), montan wax (INCI: Montan Wax), and hydrogenated castor oil isostearate; animal waxes, such as beeswax, beef tallow, beef bone fat, lard (INCI: Lard), horse fat (INCI: Horse Fat), mutton tallow, lanolin (INCI: Lanolin), insect wax, shellac wax, and sperm wax; semi-synthetic waxes, such as lanolin esters, lanolin fatty acid esters, and beeswax acid esters; hydrogenated oils, such as hydrogenated castor oil and hydrogenated coconut oil; hydrocarbon waxes, such as solid paraffin, polyethylene, ceresin, ozokerite, and microcrystalline wax; wax esters, such as synthetic beeswax; dioctyldodecyl lauroylglutamate; fatty acids, such as stearic acid and behenic acid; silicone waxes, such as acrylate silicone resins in the form of acrylate silicone graft or block copolymers (such as acrylate silicone graft copolymer: KP-561P, manufactured by Shin-Etsu Chemical Co., Ltd.); and derivatives thereof. Any one, or two or more compounds selected from the foregoing are preferable.

### • Natural animal and plant oils and semi-synthetic oils

Examples of the natural animal and plant oils and the semi-synthetic oils include naturally occurring plant oils, such as avocado oil (labeling name, INCI: Persea Gratissima (Avocado) Oil), linseed oil (labeling name, INCI: Linum Usitatissimum (Linseed) Seed Oil), almond oil (labeling name, INCI: Prunus Amygdalus Dulcis (Sweet Almond) Oil), perilla oil (labeling name), olive oil (labeling name, INCI: Olea Europaea (Olive) Fruit Oil), Torreya California oil (labeling name, INCI: Torreya Californica (California Nutmeg) Oil), citronella grass oil (labeling name, INCI: Cymbopogon Nardus (Citronella) Oil), kaya seed oil (labeling name, INCI: Torreya Nucifera Seed Oil), kyounin oil (labeling name, INCI: Kyounin Yu), wheat germ oil (labeling name, INCI: Triticum Vulgare (Wheat) Germ Oil), sesame oil (labeling name, INCI: Sesamum Indicum (Sesame) Seed Oil), rice germ oil (labeling name, INCI: Oryza Sativa (Rice) Germ Oil), rice bran oil (labeling name, INCI: Oryza Sativa (Rice) Bran Oil), camellia oil (labeling name, INCI: Camellia Kissi Seed Oil), safflower oil (labeling name, INCI: Carthamus Tinctorius (Safflower) Seed Oil), soybean oil (labeling name, INCI: Glycine Soja (Soybean) Oil), gold tea oil (labeling name, INCI: Camellia Sinensis Seed Oil), camellia oil (labeling name, INCI: Camellia Japonica Seed Oil), evening primrose oil (labeling name, INCI: Oenothera Biennis (Evening Primrose) Oil), rapeseed oil (labeling name), germ oils, such as corn germ oil (labeling name, INCI: Zea Mays (Corn) Germ Oil), persic oil (labeling name), palm oil (labeling name, INCI: Elaeis Guineensis (Palm) Oil), palm kernel oil (labeling name, INCI: Elaeis Guineensis (Palm) Kernel Oil), castor oil (labeling name, INCI: Ricinus Communis (Castor) Seed Oil), sunflower seed oil (labeling name, INCI: Helianthus Annuus (Sunflower) Seed Oil), grape seed oil (labeling name, INCI: Vitis Vinifera (Grape) Seed Oil), jojoba seed oil (labeling name, INCI: Simmondsia Chinensis (Jojoba) Seed Oil), macadamia seed oil (labeling name, INCI: Macadamia Ternifolia Seed Oil), meadowfoam oil (labeling name, INCI: Limnanthes Alba (Meadowfoam) Seed Oil), cotton seed oil (labeling name, INCI: Gossypium Herbaceum (Cotton) Seed Oil), coconut oil (labeling name, INCI: Cocos Nucifera (Coconut) Oil), and peanut oil (labeling name, INCI: Arachis Hypogaea (Peanut) Oil); natural animal oils, such as shark liver oil (labeling name, INCI: Shark Liver Oil), cod liver oil (labeling name, INCI: Cod Liver Oil), fish liver oil (labeling name, INCI: Fish Liver Oil), turtle oil (labeling name, INCI: Turtle Oil), mink oil (labeling name, INCI: Mink Oil), and egg yolk oil (labeling name, INCI: Egg Oil); and semi-synthetic oils, such as hydrogenated coconut oil (labeling name, INCI: Hydrogenated Coconut Oil) and liquid lanolin (labeling name, INCI: Lanolin Oil).

### • Hydrocarbon oils

Examples of the hydrocarbon oils include linear or branched hydrocarbon oils. The hydrocarbon oils may be volatile or nonvolatile. Specific examples include Olefin Oligomers (INCI), isoparaffins, such as (C13,C14) Isoparaffin (INCI), Isododecane (INCI), Undecane (INCI), Tridecane (INCI), Dodecane (INCI), Isohexadecane (INCI), hydrogenated polyisobutene (labeling name, INCI: Hydrogenated Polyisobutene), Squalane (INCI), Mineral Oil (INCI), and alkanes, such as Coconut Alkanes (INCI) and (C13-15) Alkane (INCI).

### • Higher alcohols

The higher alcohols include, for example, alcohols preferably having 6 or more carbon atoms, more preferably 10 to 30 carbon atoms. Specific examples of the higher alcohols include Lauryl Alcohol (INCI), Myristyl Alcohol (INCI), Palmityl Alcohol (INCI), Stearyl Alcohol (INCI), Behenyl Alcohol (INCI), Oleyl Alcohol (INCI), Isostearyl Alcohol (INCI), Octyl Dodecanol (INCI), Cholesterol (INCI), Phytosterols (INCI), and Batyl Alcohol (INCI).

### • Ester oils

Examples of the ester oils include diisobutyl adipate (labeling name, INCI: Diisobutyl Adipate), dihexyldecyl adipate (labeling name), diheptylundecyl adipate (labeling name, INCI: Diheptylundecyl Adipate), alkylglycol monoisostearates, such as isostearyl isostearate (labeling name, INCI: Isostearyl Isostearate), isocetyl isostearate (labeling name, INCI: Isocetyl Isostearate), trimethylolpropane triisostearate (labeling name, INCI: Trimethylolpropane Triisostearate), glycol diethylhexanoate (labeling name, INCI: Glycol Diethylhexanoate), cetyl ethylhexanoate (labeling name, INCI: Cetyl Ethylhexanoate), trimethylolpropane triethylhexanoate (labeling name, INCI: Trimethylolpropane Triethylhexanoate), pentaerythrityl tetraethylhexanoate (labeling name, INCI: Pentaerythrityl Tetraethylhexanoate), octyldodecyl esters, such as octyldodecyl stearoyloxystearate (labeling name, INCI: Octyldodecyl Stearoyl Stearate), oleyl oleate (labeling name, INCI: Oleyl Oleate), octyldodecyl oleate (labeling name, INCI: Octyldodecyl Oleate), decyl oleate (labeling name, INCI: Decyl Oleate), neopentyl glycol dioctanoate (labeling name, INCI: Neopentyl Glycol Diethylhexanoate), neopentyl glycol dicaprate (labeling name, INCI: Neopentyl Glycol Dicaprate), diisostearyl malate (labeling name, INCI: Diisostearyl Malate), triethyl citrate (labeling name, INCI: Triethyl Citrate), diethylhexyl succinate (labeling name, INCI: Diethylhexyl Succinate), amyl acetate (labeling name, INCI: Amyl Acetate), ethyl acetate (labeling name, INCI: Ethyl Acetate), butyl acetate (labeling name, INCI: Butyl Acetate), isocetyl stearate (labeling name, INCI: Isocetyl Stearate), butyl stearate (labeling name, INCI: Butyl Stearate), diisopropyl sebacate (labeling name, INCI: Diisopropyl Sebacate), diethylhexyl sebacate (labeling name, INCI: Diethylhexyl Sebacate), cetyl lactate (labeling name, INCI: Cetyl Lactate), myristyl lactate (labeling name, INCI: Myristyl Lactate), isononyl isononanoate (labeling name, INCI: Isononyl Isononanoate), isotridecyl isononanoate (labeling name, INCI: Isotridecyl Isononanoate), palmitates, such as isopropyl palmitate (labeling name, INCI: Isopropyl Palmitate), ethylhexyl palmitate (labeling name, INCI: Ethylhexyl Isopalmitate), and hexyldecyl palmitate (labeling name, INCI: Isocetyl Palmitate, Hexyldecyl Palmitate), cholesteryl hydroxystearate (labeling name, INCI: Cholesteryl Hydroxystearate), myristates, such as isopropyl myristate (labeling name, INCI: Isopropyl Myristate), octyldodecyl myristate (labeling name, INCI: Octyldodecyl Myristate), and myristyl myristate (labeling name, INCI: Myristyl Myristate), ethylhexyl laurate (labeling name, INCI: Ethylhexyl Laurate), hexyl laurate (labeling name, INCI: Hexyl Laurate), dioctyldodecyl lauroyl glutamate (labeling name, INCI: Dioctyldodecyl Lauroyl Glutamate), isopropyl lauroyl sarcosinate (labeling name, INCI: Isopropyl Lauroyl Sarcosinate), and cocoalkyl (caprylate/caprate) (labeling name, INCI: Coco-Caprylate·Caprate).

The ester oils include glyceride oils, such as triethylhexanoin (labeling name, INCI:), glyceryl tri(caprylate/caprate) (labeling name, INCI: Caprylic/Capric Triglyceride), Cocoglyceryl (INCI), triglyceryl (caprylate/caprate/succinate) (labeling name, INCI: Caprylic/Capric/Succinic Triglyceride), and (caprylic/capric) glycerides (labeling name, INCI: Caprylic/Capric Glycerides).

### • Fluorinated oils

Examples of the fluorinated oils include Perfluorodecalin (INCI), Perfluorononyl Dimethicone (INCI), and Perfluoromethylcyclopentane (INCI).

### • UV absorbers

Suitable UV absorbers include oxybenzone-1 (labeling name, INCI: Benzophenone-1), oxybenzone-2 (labeling name, INCI: Benzophenone-2), oxybenzone-3 (labeling name, INCI: Benzophenone-3), oxybenzone-4 (labeling name, INCI: Benzophenone-4), oxybenzone-5 (labeling name, INCI: Benzophenone-5), oxybenzone-6 (labeling name, INCI: Benzophenone-6), oxybenzone-9 (labeling name, INCI: Benzophenone-9), Homosalate (INCI), Octocrylene (INCI), t-butyl methoxydibenzoylmethane (labeling name, INCI: Butyl Methoxydibenzoylmethane), ethylhexyl salicylate (labeling name, INCI: Ethylhexyl Salicylate), diethylaminohydroxybenzoyl hexyl benzoate (labeling name, INCI: Diethylamino Hydroxybenzoyl Hexyl Benzoate), Polysilicone-15 (INCI), dimethoxybenzylidene dioxoimidazolidine octyl propionate (labeling name, INCI: Ethylhexyl Dimethoxybenzylidene Dioxoimidazolidine Propionate), terephthalylidene dicamphor sulfonic acid (labeling name, INCI: Terephthalylidene Dicamphor Sulfonic Acid), Ethylhexyl Triazone (INCI), methylbis(trimethylsiloxy)silyl isopentyl trimethoxycinnamate (labeling name, INCI: Isopentyl Trimethoxycinnamate Trisiloxane), Drometrizole Trisiloxane (INCI), dimethyl PABA ethylhexyl (labeling name, INCI: Ethylhexyl Dimethyl PABA), isopropyl para-methoxycinnamate (labeling name, INCI: Isopropyl Methoxycinnamate), ethylhexyl methoxycinnamate (labeling name, INCI: Ethylhexyl Methoxycinnamate), Bis-Ethylhexyloxyphenol Methoxyphenyltriazine (INCI), phenylbenzimidazole sulfonic acid (labeling name, INCI: Phenylbenzimidazole Sulfonic Acid), Methylene Bis-Benzotriazolyl Tetramethylbutylphenol (INCI), glyceryl ethylhexanoate dimethoxycinnamate (labeling name, INCI: Glyceryl Ethylhexanoate Dimethoxycinnamate), Glyceryl PABA (INCI), methyl diisopropylcinnamate (labeling name, INCI: Diisopropyl Methyl Cinnamate), Cinoxate (INCI), and ethylhexyl dimethoxybenzylidene dioxoimidazolidine propionate (labeling name, INCI: Ethylhexyl Dimethoxybenzylidene Dioxoimidazolidine Propionate).

### (2) Aqueous components other than the components (b) and (d)

The aqueous components are not particularly limited as long as they do not belong to the components (b) or (d) and are commonly blended in cosmetics. Specific examples include humectants, such as Betaine (INCI), PCA-Na (labeling name, INCI: Sodium PCA), egg yolk lecithin, soybean lecithin, phosphatidylcholine, phosphatidylethanolamine, phosphatidylserine, and sphingophospholipid. Examples further include water-soluble polymer compounds, for example, gum Arabic, guar gum, carrageenan, agar, quince seed gum, locust bean gum, xanthan gum, pullulan, sodium carboxymethyl cellulose, hydroxyethyl cellulose, vinyl polymers, such as carboxyvinyl polymer, and acrylic polymers, such as (ammonium acryloyldimethyl taurate/vinylpyrrolidone) copolymer, (sodium acrylate/sodium acryloyldimethyl taurate) copolymer, (hydroxyethyl acrylate/sodium acryloyldimethyl taurate) copolymer, (acrylamide/sodium acryloyldimethyl taurate) copolymer, and polyacrylamide. In particular, oil-in-water cosmetics can be stabilized in a relatively easy manner by using an acrylic polymer.

### (3) Surfactants other than the components (c)

The surfactants may be nonionic, anionic, cationic, or ampholytic. The surfactants are not particularly limited as long as they do not belong to the components (c) and are commonly blended in cosmetics. Of the surfactants, one, or two or more surfactants selected from non-crosslinked silicone surfactants and crosslinked silicone surfactants are preferred for the reason that a stable cosmetic can be obtained. In any case where the surfactant other than the components (c) is added, the amount thereof is preferably 0.1 to 20% by mass of the cosmetic. An amount of at least 0.1% by mass is preferred in that the dispersing and emulsifying functions are fully achieved whereas an amount of up to 20% by mass is preferred because the risk that the cosmetic gives a sticky feeling on use is eliminated. The surfactant other than the components (c) preferably has an HLB of 2 to 14.5, though not limitative, for the purpose of maintaining the water resistance of the cosmetic.

The non-crosslinked silicone surfactants are linear or branched silicones in which some methyl groups on the backbone are substituted with hydrophilic groups, such as polyethylene glycol and polyglycerin. Specifically, preferred such surfactants are linear or branched polyoxyethylene-modified organopolysiloxanes, linear or branched polyoxyethylene polyoxypropylene-modified organopolysiloxanes, linear or branched polyoxyethylene·alkyl-co-modified organopolysiloxanes, linear or branched polyoxyethylene polyoxypropylene·alkyl-co-modified organopolysiloxanes, linear or branched polyglycerin-modified organopolysiloxanes, linear or branched polyglycerin·alkyl-co-modified organopolysiloxanes, and linear or branched pyrrolidone-modified organopolysiloxanes. Specific examples include PEG-11 Methyl Ether Dimethicone (INCI), PEG/PPG-20/22 Butyl Ether Dimethicone (INCI), PEG-3 Dimethicone (INCI), PEG-10 Dimethicone (INCI), PEG-9 Polydimethylsiloxyethyl Dimethicone (INCI), Lauryl PEG-9 Polydimethylsiloxyethyl Dimethicone (INCI), Cetyl PEG/PPG-10/1 Dimethicone (INCI), Polyglyceryl-3 Polydimethylsiloxyethyl Dimethicone (INCI), Lauryl Polyglyceryl-3 Polydimethylsiloxyethyl Dimethicone (INCI), and Isostearyl Polyglyceryl-3 Dimethicone (INCI).

Commercially available examples include KF-6011, KF-6011P, KF-6012, KF-6015, KF-6017, KF-6043, KF-6028, KF-6038, KF-6048, KF-6104, KF-6106, KF-6105, KF-6180, and KF-6115, manufactured by Shin-Etsu Chemical Co., Ltd.

Examples of the crosslinked silicone surfactants include (Dimethicone/(PEG-10/15)) Crosspolymer (INCI), (PEG-15/Lauryl Dimethicone) Crosspolymer (INCI), (PEG-10/Lauryl Dimethicone) Crosspolymer (INCI), (PEG-15/Lauryl Polydimethylsiloxyethyl Dimethicone) Crosspolymer (INCI), (Dimethicone/Polyglycerin-3) Crosspolymer (INCI), (Lauryl Dimethicone/Polyglycerin-3) Crosspolymer (INCI), and (Polyglycerin-3/Lauryl Polydimethylsiloxyethyl Dimethicone) Crosspolymer (INCI).

On use of the crosslinked silicone surfactant, it is preferable that in a composition consisting of the crosslinked silicone surfactant and an oil that is liquid at room temperature, the crosslinked silicone surfactant swell with the liquid oil as a result of being impregnated with an amount of more than its own weight of the liquid oil.

Suitable liquid oils include liquid silicone oils, hydrocarbon oils, ester oils, natural animal and plant oils, semi-synthetic oils, and fluorinated oils, all exemplified above as the oils (1) that are optional components. Illustrative examples include Cyclopentasiloxane (INCI), Dimethicone (INCI), Caprylyl Methicone (INCI), Mineral Oil (INCI), Isododecane (INCI), Isohexadecane (INCI), Triethylhexanoin (INCI), isotridecyl isononanoate (labeling name, INCI: Isotridecyl Isononanoate), and Squalane (INCI).

Commercially available examples of the crosslinked silicone surfactants that are swollen with the liquid oil include KSG-210, KSG-240, KSG-270, KSG-310, KSG-320, KSG-330, KSG-340, KSG-320Z, KSG-350Z, KSG-710, KSG-790, KSG-810, KSG-820, KSG-830, KSG-840, KSG-820Z, and KSG-850Z from Shin-Etsu Chemical Co., Ltd.

### (4) Powders other than the components (a) and (e)

Suitable powders other than the components (a) include coloring pigments, inorganic powders, metal powders, organic powders, and inorganic/organic composite powders. Specific examples include the following.

### • Coloring pigments

The coloring pigments are not particularly limited as long as they are commonly used in cosmetics for the purpose of coloring. Examples include red iron oxide (labeling name, INCI: Iron Oxides), yellow iron oxide (labeling name, INCI: Iron Oxides), white titanium dioxide (labeling name, INCI: Titanium Dioxide), black iron oxide (labeling name, INCI: Iron Oxides), Ultramarine (labeling name, INCI: Ultramarines), Prussian blue (labeling name, INCI: Ferric Ferrocyanide, Ferric Ammonium Ferrocyanide), manganese violet (labeling name, INCI: Manganese Violet), cobalt titanate (labeling name, INCI: Cobalt Titanium Oxide), chromium hydroxide (labeling name, INCI: Chromium Hydroxide Green), chromium oxide (labeling name, INCI: Chromium Oxide Greens), Al/cobalt oxide (labeling name, INCI: Cobalt Aluminum Oxide), fired titanium/titanium dioxide (labeling name, INCI: Titanium/Titanium Dioxide), Li/cobalt titanate (labeling name, INCI: Lithium Cobalt Titanate), sintered iron oxide/titanium dioxide (labeling name), composites doped with a different metal, such as iron oxide-doped titanium dioxide (labeling name, INCI: Iron Oxides, Titanium Dioxide), titanium nitride (labeling name, INCI: Titanium Nitride), ferrous hydroxide (labeling name, INCI: Iron Hydroxide), inorganic brown pigments, such as γ-iron oxide, inorganic yellow pigments, such as loess, and colored pigments, such as lake form of tar dyes and lake form of natural dyes. Any of the foregoing may be used.

The pigment may take any shape including spherical, generally spherical, bar, spindle, petal, strip, and irregular shapes. Its geometry is not particularly limited as long as a color can be imparted to the cosmetic.

### • Inorganic powders

Examples of the inorganic powders include fine particles of zirconium oxide (labeling name, INCI: Zirconium Dioxide), zinc oxide (labeling name, INCI: Zinc Oxide), cerium oxide (labeling name, INCI: Cerium Oxide), magnesium oxide (labeling name, INCI: Magnesium Oxide), barium sulfate (labeling name, INCI: Barium Sulfate), calcium sulfate (labeling name, INCI: Calcium Sulfate), magnesium sulfate (labeling name, INCI: Magnesium Sulfate), calcium carbonate (labeling name, INCI: Calcium Carbonate), magnesium carbonate (labeling name, INCI: Magnesium Carbonate), Talc (INCI), Mica (INCI), Kaolin (INCI), synthetic fluorphlogopite (labeling name, INCI: Synthetic Fluorphlogopite), synthetic ferrous golden mica (labeling name), black mica (labeling name, INCI: Biotite), potassium silicate (labeling name, INCI: Potassium Silicate), Silica (INCI), aluminum silicate (labeling name, INCI: Aluminum Silicate), magnesium silicate (labeling name, INCI: Magnesium Silicate), Al/Mg silicate (labeling name, INCI: Magnesium Aluminum Silicate), calcium silicate (labeling name, INCI: Calcium Silicate), Al/Ca/Na silicate (labeling name, INCI: Aluminum Calcium Sodium Silicate), Li/Mg/Na silicate (labeling name, INCI: Lithium Magnesium Sodium Silicate), Na/Mg silicate (labeling name, INCI: Sodium Magnesium Silicate), Ca/Al borosilicate (labeling name, INCI: Calcium Aluminum Borosilicate), Ca/Na borosilicate (labeling name, INCI: Calcium Sodium Borosilicate), Hydroxyapatite (INCI), Bentonite (INCI), Montmorillonite (INCI), Hectorite (INCI), Zeolite (INCI), Alumina (INCI), Al hydroxide (labeling name, INCI: Aluminum Hydroxide), boron nitride (labeling name, INCI: Boron Nitride), and glass (labeling name, INCI: Glass).

Also, examples of the inorganic coloring pearly pigments include pearly agents, such as Mica (INCI) coated with titanium dioxide (labeling name, INCI: Titanium Dioxide), and synthetic fluorphlogopite (labeling name, INCI: Synthetic Fluorphlogopite) coated with titanium dioxide (labeling name, INCI: Titanium Dioxide); and pearly pigments, such as bismuth oxychloride (labeling name, INCI: Bismuth Oxychloride), bismuth oxychloride (labeling name, INCI: Bismuth Oxychloride) coated with titanium dioxide (labeling name, INCI: Titanium Dioxide), Talc (INCI) coated with titanium dioxide (labeling name, INCI: Titanium Dioxide), fish scale flakes (labeling name), and coloring mica coated with titanium dioxide (labeling name, INCI: Titanium Dioxide). These may either be untreated or have undergone well-known surface treatment commonly used for cosmetics.

### • Metal powders

Examples of the metal powders include fine particles of metals, such as aluminum (labeling name, INCI: Aluminum, Aluminum Powder), copper (labeling name, INCI: Copper Powder), silver (labeling name, INCI: Silver Powder), and gold (labeling name, INCI: Gold).

### • Organic powders

Examples of the organic powders include powders of silicones, polyamides, polyacrylic acid-acrylate, polyesters, Polyethylene (INCI), Polypropylene (INCI), Polystyrene (INCI), styrene-acrylic acid copolymers, divinylbenzene-styrene copolymers, polyurethanes, vinyl resins, urea resins, melamine resins, benzoguanamine, polymethyl benzoguanamine, tetrafluoroethylene, polymethyl methacrylate, Cellulose (INCI), Silk (INCI), nylon (labeling name), phenolic resins, epoxy resins, and polycarbonate.

In particular, examples of the silicones include silicone resin particles; Polymethylsilsesquioxane (INCI), silicone rubber powder, silicone resin-coated silicone rubber powder; (vinyl dimethicone/methicone silsesquioxane) crosspolymer (labeling name, INCI: Vinyl Dimethicone/Methicone Silsesquioxane Crosspolymer), (diphenyl dimethicone/vinyl diphenyl dimethicone/silsesquioxane) crosspolymer (labeling name, INCI: Diphenyl Dimethicone/Vinyl Diphenyl Dimethicone/Silsesquioxane Crosspolymer), Polysilicone-1 Crosspolymer (INCI), and Polysilicone-22 (INCI).

Commercially available examples of the silicone powders include KMP-590, KMP-591, KMP-592, KMP-597, KMP-598, KSP-100, KSP-101, KSP-102, KSP-105, KSP-100W, KSP-300, KSP-411, KSP-441, KM-9729, and KM-440, from Shin-Etsu Chemical Co., Ltd.

Examples further include metal soaps. Specific examples include powders of zinc stearate (labeling name, INCI: Zinc Stearate), Al stearate (labeling name, INCI: Aluminum Stearate), Ca stearate (labeling name, INCI: Calcium Stearate), Mg stearate (labeling name, INCI: Magnesium Stearate), zinc myristate (labeling name, INCI: Zinc Myristate), Mg myristate (labeling name, INCI: Magnesium Myristate), Zn/Na cetyl phosphate (labeling name, INCI: Sodium Zinc Cetyl Phosphate), and K cetyl phosphate (labeling name, INCI: Potassium Cetyl Phosphate).

Examples further include organic colorants. Specific examples include tar dyes, such as Red #3, Red #104 (1) (labeling name, INCI: Red 28, Red 28 Lake), Red #106, Red #201 (labeling name, INCI: Red 6), Red #202 (labeling name, INCI: Red 7), Red #204, Red #205, Red #220 (labeling name, INCI: Red 34), Red #226 (labeling name, INCI: Red 30), Red #227 (labeling name, INCI: Red 33, Red 33 Lake), Red #228 (labeling name, INCI: Red 36), Red #230 (1) (labeling name, INCI: Red 22, Red 22 Lake), Red #230 (2) (labeling name), Red #401 (labeling name), Red #505 (labeling name), Yellow #4 (labeling name, INCI: Yellow 5), Yellow #5 (labeling name, INCI: Yellow 6, Yellow 6 Lake), Yellow #202 (1) (labeling name, INCI: Yellow 8), Yellow #203 (labeling name, INCI: Yellow 10, Yellow 10 Lake), Yellow #204 (labeling name, INCI: Yellow 11), Yellow #401, Blue #1 (labeling name, INCI: Blue 1, Blue 1 Lake), Blue #2, Blue #201, Blue #205 (labeling name, INCI: Blue 4), Blue #404 (labeling name), Green #3 (labeling name, INCI: Green 3, Green 3 Lake), Green #201 (labeling name, INCI: Green 5), Green #202 (labeling name, INCI: Green 6), Green #204 (labeling name, INCI: Green 8), Green #205 (labeling name), Orange #201 (labeling name, INCI: Orange 5), Orange #203 (labeling name, INCI: Pigment Orange 5), Orange #204 (labeling name), Orange #205 (labeling name, INCI: Orange 4, Orange 4 Lake), Orange #206 (labeling name, INCI: Orange 10), and Orange #207 (labeling name, INCI: Orange 11); and natural dyes, such as Cochineal (INCI), laccaic acid (labeling name, INCI: Laccaic Acid), safflower red (labeling name, INCI: Carthamus Tinctorius (Safflower) Flower Extract), red root extract (labeling name, INCI: Lithospermum Officinale Root Extract), gardenia yellow (labeling name), and gardenia blue (labeling name, INCI: Hydrolyzed Gardenia Florida Extract).

### • Inorganic/organic composite powders

Examples of the inorganic/organic composite powders include composite powders obtained by coating the surface of an inorganic powder with an organic powder by any well-known method.

The powders described above may be used as having been treated on the surface of particles. The surface treating agent is preferably one capable of imparting hydrophobicity from the point of view of water resistance of the cosmetic. The hydrophobizing agent is not particularly limited. Exemplary treating agents include silicone treating agents, waxes, paraffins, organic fluorine compounds, such as perfluoroalkyl phosphate salts, surfactants, amino acids, such as N-acylglutamic acid, and metal soaps, such as aluminum stearate and magnesium myristate.

Silicone treating agents are more preferable. Examples include silanes or silylating agents, such as Triethoxycaprylylsilane (INCI); Dimethicone (INCI), Methicone (INCI), Hydrogen Dimethicone (INCI), Triethoxysilylethyl Polydimethylsiloxyethyl Dimethicone (INCI), Triethoxysilylethyl Polydimethylsiloxyethylhexyl Dimethicone (INCI), and acrylates/tridecyl acrylate/triethoxysilylpropyl methacrylate/dimethicone methacrylate copolymer (labeling name, INCI: Acrylates/Tridecyl Acrylate/Triethoxysilylpropyl Methacrylate/Dimethicone Methacrylate Copolymer).

Specific examples of the silicone treating agents include AES-3083, KF-99P, KF-9901, KF-9908, KF-9909, KP-574, and KP-541, from Shin-Etsu Chemical Co., Ltd.

The surface hydrophobizing agents may be used singly, or two or more may be used in combination. Specific examples of the surface-treated coloring pigments include KTP-09 series, especially KTP-09W, KTP-09R, KTP-09Y, and KTP-09B, from Shin-Etsu Chemical Co., Ltd.

### • UV absorbing/scattering agents

In addition to the aqueous dispersion of the present invention, a dispersion of the component (a) and/or the component (e), namely, UV absorbing/scattering particles, previously dispersed in an oil may also be used.

Suitable oils include liquid silicone oils, hydrocarbon oils, ester oils, natural animal and plant oils, semi-synthetic oils, and fluorinated oils, all exemplified above as the oils (1) that are optional components.

Specific examples of the dispersions of UV absorbing/scattering particles previously dispersed in an oil include SPD series (product name), especially SPD-T5, SPD-T5L, SPD-Z5, SPD-T6, SPD-Z6, SPD-T7, and SPD-Z7L, from Shin-Etsu Chemical Co., Ltd.

### • Hydrophilized UV-absorbing/scattering agents

Hydrophilized fine particles of titanium dioxide and hydrophilized fine particles of zinc oxide may be used in addition to the components (a) and (e) in the present invention. Specific examples of the hydrophilic treatments include treatments with hydrous silica and treatments with high-HLB surfactants. The hydrophilized UV-scattering agents have poor water resistance, and are therefore not suitable for cosmetics that require water resistance.

### (5) Compositions consisting of a crosslinked organopolysiloxane and an oil that is liquid at room temperature

In the composition consisting of a crosslinked organopolysiloxane and an oil that is liquid at room temperature, it is preferable that the crosslinked organopolysiloxane swell with the liquid oil as a result of being impregnated with an amount of more than its own weight of the liquid oil. Suitable liquid oils include liquid silicone oils, hydrocarbon oils, ester oils, natural animal and plant oils, semi-synthetic oils, and fluorinated oils, all exemplified above as the oils (1) that are optional components. Illustrative examples include Cyclopentasiloxane (INCI), Dimethicone (INCI), Mineral Oil (INCI), Isododecane (INCI), Isohexadecane (INCI), Triethylhexanoin (INCI), isotridecyl isononanoate (labeling name, INCI: Isotridecyl Isononanoate), Squalane (INCI), and cocoalkyl (caprylate/caprate) (labeling name, INCI: Coco-Caprylate/Caprate).

Unlike the crosslinked silicone surfactants as the components (3) mentioned above, the components (5) are compounds having neither a polyether nor a polyglycerin structure in the molecular structure. Specific examples include Dimethicone/Vinyl Dimethicone Crosspolymer (INCI), dimethicone/phenyl vinyl dimethicone crosspolymer (labeling name, INCI: Dimethicone/Phenyl Vinyl Dimethicone Crosspolymer), Vinyl Dimethicone/Lauryl Dimethicone Crosspolymer (INCI), and Lauryl Polydimethylsiloxyethyl Dimethicone/Bis-Vinyl Dimethicone Crosspolymer (INCI).

Commercially available examples of the compositions consisting of a crosslinked organopolysiloxane and an oil that is liquid at room temperature include KSG-15, KSG-1510, KSG-16, KSG-1610, KSG-19, KSG-016F, KSG-18A, KSG-41A, KSG-42A, KSG-43, KSG-44, KSG-45, KSG-042Z, KSG-045Z, KSG-048Z, and KM-116, from Shin-Etsu Chemical Co., Ltd.

### (6) Film-forming agents

The film-forming agent is added mainly for the purpose of further maintaining the effect-sustainability of the cosmetic. A silicone-based composition is preferable from the point of view of imparting water repellency, though the agent is not limited thereto. Specifically, use may be made of, for example, trimethylsiloxysilicate, acrylic-silicone film formers, silicone-modified norbornene, silicone-modified pullulan, and silicone-modified polyvinyl alcohol.

Examples of the film-forming agents in the form of silicone-based composition include trimethylsiloxysilicate (labeling name, INCI: Trimethylsiloxysilicate), Acrylates/Dimethicone Copolymer (INCI), Norbornene/Tris-(Trimethylsiloxy)Silylnorbornene Copolymer (INCI), and pullulan tri(trimethylsiloxy)silylpropyl carbamide (labeling name, INCI: Trimethylsiloxysilylcarbamoyl Pullulan).

The film-forming agent may be previously dissolved in an oil that is liquid at room temperature before it is added in the cosmetic. Suitable liquid oils used herein include liquid silicone oils, hydrocarbon oils, ester oils, natural animal and plant oils, semi-synthetic oils, and fluorinated oils, all exemplified above as the oils (1) that are optional components. Specific examples of commercially available silicone film-forming agents include KF-7312J, KP-545, KP-549, KP-543, NBN-30-ID, TSPL-30-ID, and TSPL-30-D5, from Shin-Etsu Chemical Co., Ltd.

### (7) Other additives

Other additives include oil-soluble gelling agents, preservatives, antibacterial agents, antiperspirants, fragrances, salts, antioxidants, acidity regulators, chelating agents, refreshing agents, anti-inflammatory agents, skin modifying ingredients (e.g., brightening or whitening agents, cell activators, anti-skin-roughening agents, blood flow enhancing agents, skin astringents, antiseborrheic agents), vitamins, amino acids, nucleic acids, hormones, and clathrate compounds.

### • Oil-soluble gelling agents

Exemplary oil-soluble gelling agents include metal soaps, such as aluminum stearate, magnesium stearate, and zinc myristate; amino acid derivatives, such as lauroyl glutamic acid (labeling name, INCI: Lauroyl Glutamic Acid) and α,γ-di-n-butylamine; dextrin fatty acid esters, such as dextrin palmitate (labeling name, INCI: Dextrin Palmitate), dextrin isostearate (labeling name, INCI: Dextrin Isostearate), dextrin myristate (labeling name, INCI: Dextrin Myristate), inulin stearate (labeling name, INCI: Stearoyl Inulin), and dextrin palmitate/ethylhexanoate (labeling name, INCI: Dextrin Palmitate/Ethylhexanoate); sucrose fatty acid esters, such as sucrose palmitate and sucrose stearate; fructooligosaccharide fatty acid esters, such as fructooligosaccharide stearate and fructooligosaccharide 2-ethylhexanoate; benzylidene derivatives of sorbitol, such as monobenzylidene sorbitol and dibenzylidene sorbitol; organo-modified clay minerals, such as Disteardimonium Hectorite (INCI), Stearalkonium Hectorite (INCI), and hectorite; and Stearalkonium Bentonite (INCI).

### • Preservatives/antibacterial agents

Suitable preservatives and antibacterial agents include alkyl p-hydroxybenzoates, benzoic acid, sodium benzoate, sorbic acid, potassium sorbate, phenoxyethanol, imidazolidinyl urea, salicylic acid, isopropyl methyl phenol, phenol, p-chloro-m-cresol, hexachlorophene, benzalkonium chloride, chlorohexidine chloride, trichlorocarbanilide, iodopropynyl butylcarbamate, polylysine, bisabolol, ethylhexylglycerin, glyceryl caprylate, caprylhydroxamic acid, hexyl dimethylolpropanoate, polyaminopropyl biguanide, photosensitizers, silver, and plant extracts. Preservatives may be used singly, or two or more may be used in combination. In particular, polylysine, bisabolol, ethylhexylglycerin, glyceryl caprylate, caprylhydroxamic acid, hexyl dimethylolpropanoate, polyaminopropyl biguanide, ethylhexylglycerin, and phenoxyethanol are preferable because they are easily blended into the cosmetic and are expected to produce preservative effects. Ethylhexylglycerin, phenoxyethanol, and glyceryl caprylate are particularly preferable in terms of the compatibility with the dispersion of the present invention. The addition of preservatives is expected to suppress bacterial contamination and enhance the storage stability of the cosmetic.

### • Antiperspirants

Examples of the antiperspirants include aluminum halohydrates, such as aluminum chlorohydrate, aluminum halides, such as aluminum chloride, aluminum allantoinate, tannic acid, persimmon tannin, aluminum potassium sulfate, zinc oxide, zinc p-phenolsulfonate, aluminum potassium sulfate, aluminum zirconium tetrachlorohydrate, and aluminum zirconium trichlorohydrex glycine. The preferred components that exert a higher effect include aluminum halohydrates, aluminum halides, and complexes or mixtures thereof with zirconium oxyhalides and zirconium hydroxyhalides (e.g., aluminum zirconium tetrachlorohydrate and aluminum zirconium trichlorohydrex glycine).

### • Fragrances

Examples of the fragrances include natural fragrances and synthetic fragrances. Suitable natural fragrances include plant fragrances separated from flowers, leaves, stems, pericarps, and the like; and animal fragrances, such as musk and civet. Suitable synthetic fragrances include hydrocarbons, such as monoterpene; alcohols, such as aliphatic alcohols and aromatic alcohols; aldehydes, such as terpene aldehydes and aromatic aldehydes; ketones, such as alicyclic ketones; esters, such as terpene esters; lactones; phenols; oxides; nitrogen-containing compounds; and acetals.

### • Salts

Examples of the salts include inorganic salts, organic acid salts, amine salts, and amino acid salts. Exemplary inorganic salts include sodium salts, potassium salts, magnesium salts, calcium salts, aluminum salts, zirconium salts, and zinc salts of inorganic acids, such as hydrochloric acid, sulfuric acid, carbonic acid, and nitric acid. Exemplary organic acid salts include salts of organic acids, such as acetic acid, dehydroacetic acid, citric acid, malic acid, succinic acid, ascorbic acid, and stearic acid. Exemplary amine salts and amino acid salts include salts of amines, such as triethanolamine, and salts of amino acids, such as glutamic acid. Also useful are salts of hyaluronic acid, chondroitin sulfate, aluminum zirconium glycine complex, and acid-alkali neutral salts used in cosmetic formulations. In particular, sodium chloride is preferred from the aspects of dissolution, feeling on use, and anti-foaming. When used in a large amount, the salt can inhibit the swelling of a water-soluble polymer compound.

### • Antioxidants

Examples of the antioxidants include, but are not particularly limited to, carotenoid, ascorbic acid and salts thereof, ascorbyl stearate, tocophenol, tocopherol acetate, tocopherol, p-t-butylphenol, butyl hydroxyanisole, dibutyl hydroxytoluene, phytic acid, ferulic acid, thiotaurine, hypotaurine, sulfites, erythorbic acid and salts thereof, chlorogenic acid, epicatechin, epigallocatechin, epigallocatechin gallate, apigenin, kaempferol, myricetin, and quercetin. The antioxidants may be used singly, or two or more may be used in combination.

### • Acidity regulators

Suitable acidity regulators include lactic acid, citric acid, glycolic acid, succinic acid, tartaric acid, dl-malic acid, potassium carbonate, sodium hydrogencarbonate, and ammonium hydrogencarbonate.

### • Chelating agents

Suitable chelating agents include alanine, sodium EDTA, sodium polyphosphate, sodium metaphosphate, and phosphoric acid.

### • Refreshing agents

Suitable refreshing agents include L-menthol, camphor, and menthyl lactate.

### • Anti-inflammatory agents

Suitable anti-inflammatory agents include allantoin, glycyrrhizinic acid and salts thereof, glycyrrhetinic acid, stearyl glycyrrhetinate, tranexamic acid, and azulene.

### • Skin modifying ingredients

Suitable skin modifying ingredients include brightening or whitening agents, such as placenta extract, arbutin, glutathione, and Saxifraga Sarmentosa Extract; cell activators, such as royal jelly, photosensitizers, cholesterol derivatives, and bovine blood extracts; anti-skin-roughening agents; blood flow enhancing agents, such as vanillylamide nonanoate, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-oryzanol; skin astringents, such as zinc oxide and tannic acid; and antiseborrheic agents, such as sulfur and thianthol.

### • Vitamins

Suitable vitamins include vitamin A family, such as vitamin A oil, retinol, retinol acetate, and retinol palmitate; vitamin B family, specifically, vitamin B2 family, such as riboflavin, riboflavin butyrate, and flavin adenine nucleotide, vitamin B6 family, such as pyridoxine hydrochloride, pyridoxine dioctanoate, and pyridoxine tripalmitate, vitamin B12 and derivatives thereof, and vitamin B15 and derivatives thereof; vitamin C family, such as L-ascorbic acid, L-ascorbic acid dipalmitic acid ester, L-ascorbic acid-2-sodium sulfate, and dipotassium L-ascorbic acid phosphoric acid diester; vitamin D family, such as ergocalciferol and cholecalciferol; vitamin E family, such as α-tocopherol, β-tocopherol, γ-tocopherol, dl-α-tocopherol acetate, dl-α-tocopherol nicotinate, and dl-α-tocopherol succinate; nicotinic acids, such as nicotinic acid, benzyl nicotinate, and nicotinic amide; vitamin H; vitamin P; pantothenic acids, such as calcium pantothenate, D-pantothenyl alcohol, pantothenyl ethyl ether, and acetylpantothenyl ethyl ether; and biotin.

### • Amino acids

Suitable amino acids include glycine, valine, leucine, isoleucine, serine, threonine, phenylalanine, arginine, lysine, aspartic acid, glutamic acid, cystine, cysteine, methionine, and tryptophan.

### • Nucleic acids

Typical of the nucleic acids is deoxyribonucleic acid.

### • Hormones

Suitable hormones include estradiol and ethinyl estradiol.

### • Clathrate compounds

Typical of the clathrate compounds is cyclodextrin.

### EXAMPLES

The present invention will be described in detail below by presenting Examples and Comparative Examples. However, the present invention is not limited to the following Examples. In the examples below, the compositional percent "%" and the ratio are by mass unless otherwise stated. The amount of product name is the amount of the product added. The mass ratio (c)/(b) is the ratio of pure components.

### [Whether or not the component (c) dissolves]

The components described below were each mixed with water or BG so that their concentration would be 20% by mass, and then allowed to stand at 25°C for 1 hour. The rating was "dissolve" when the mixture was transparent to semi-transparent without any boundary. The rating was "not dissolve" when the mixture had become white turbid or had separated into two layers. Being "transparent to semi-transparent" means that the total light transmission through a 1 cm thick cell filled with the mixture liquid is 50% or more as measured in accordance with the method described in JIS K7361-1: 1997.

**[Table 1]**

| | Solubility in water | Solubility in BG |
|---|---|---|
| (c) KF-6100 (Note 1) | Not dissolve | Dissolve |
| KF-6106 (Note 2) | Not dissolve | Not dissolve |

| | |
|---|---|
| (Note 1) | Polyglyceryl-3 disiloxane dimethicone (labeling name, INCI: Polyglyceryl-3 Disiloxane Dimethicone) |
| (Note 2) | Polyglyceryl-3 polydimethylsiloxyethyl dimethicone (labeling name, INCI: Polyglyceryl-3 Polydimethylsiloxyethyldimethicone) |

### [Examples for dispersions]

Dispersions were obtained by preparing slurries based on the formulations described in Table 2 below using a roll mill. The dispersions obtained were evaluated for dispersibility according to the evaluation criteria described below. The results are also described in the table.

### [Dispersibility in water]

The dispersions in the table below were each collected in an amount of 0.5 g with a metal spatula and added to a beaker containing 50 mL of water. Stirring was then performed to evaluate the dispersibility in water.
A: The dispersion was dispersed uniformly with no sedimentation.
B: The dispersion showed temporary sedimentation, but became uniformly dispersed upon continued stirring.
C: The dispersion was not dispersed.

The sample was judged "Pass" when rated with "B" or above.

The dispersions in the examples that were pastes had a hardness of 1 to 100, and those that were liquid had a viscosity of 50 to 5,000 mPa·s. The hardness is a value measured using Rheometer RT-2002D·D (manufactured by RHEOTECH, probe: 5 mmφ, penetration depth: 10 mm, sample stage rising speed: 5 cm/min, temperature: 25°C, range: 200). The viscosity is a value measured at 25°C using a Brookfield viscometer (TVB-10, manufactured by Toki Sangyo Co., Ltd.) in accordance with the method described in JIS K7117-1: 1999.

**[Table 2]**

| Formulation (%) | Example | | | | | Comparative Example | | | |
|---|---|---|---|---|---|---|---|---|---|
| | 1 | 2 | 3 | 4 | 5 | 1 | 2 | 3 | 4 |
| (a) FINEX-52W-LP2 (Note 1) | 65 | - | 70 | 62 | - | 70 | 65 | 65 | 65 |
| (a) FINEX-33W-LP2 (Note 2) | - | - | - | - | 60 | - | - | - | - |
| (a) FINEX-50LP (Note 3) | - | 65 | - | - | - | - | - | - | - |
| (b) BG | 25 | 25 | 25 | 20 | 25 | - | - | 35 | 25 |
| Ethanol | - | - | - | - | - | 25 | - | - | - |
| (c) KF-6100 (Note 4) | 10 | 10 | 5 | 18 | 15 | 5 | 35 | - | - |
| KF-6106 (Note 5) | - | - | - | - | - | - | - | - | 10 |
| Total | 100 | 100 | 100 | 100 | 100 | 100 | 100 | 100 | 100 |
| (c)/(b) | 0.4 | 0.4 | 0.2 | 0.9 | 0.6 | - | - | - | - |
| Dispersibility in water | A | B | B | B | B | C | C | C | C |

| | |
|---|---|
| (Note 1) | Fine particles of zinc oxide (labeling name, INCI: Zinc Oxide) treated with hydrous silica (labeling name, INCI: Hydrated Silica) and with hydrogen dimethicone (labeling name, INCI: Hydrogen Dimethicone), number average primary particle diameter determined by image analysis of a transmission electron photomicrograph: 20 nm (average diameter of 200 particles measured by image analysis of a transmission electron photomicrograph) |
| (Note 2) | Fine particles of zinc oxide (labeling name, INCI: Zinc Oxide) treated with hydrous silica (labeling name, INCI: Hydrated Silica) and with hydrogen dimethicone (labeling name, INCI: Hydrogen Dimethicone), number average primary particle diameter determined by image analysis of a transmission electron photomicrograph: 35 nm (average diameter of 200 particles measured by image analysis of a transmission electron photomicrograph) |
| (Note 3) | Fine particles of zinc oxide (labeling name, INCI: Zinc Oxide) treated with hydrogen dimethicone (labeling name, INCI: Hydrogen Dimethicone), number average primary particle diameter determined by image analysis of a transmission electron photomicrograph: 35 nm (average diameter of 200 particles measured by image analysis of a transmission electron photomicrograph) |
| (Note 4) | Polyglyceryl-3 disiloxane dimethicone (labeling name, INCI: Polyglyceryl-3 Disiloxane Dimethicone) |
| (Note 5) | Polyglyceryl-3 polydimethylsiloxyethyl dimethicone (labeling name, INCI: Polyglyceryl-3 Polydimethylsiloxyethyldimethicone) |

As is clear from Table 2, the dispersions (slurries) obtained in Examples of the present invention achieved good dispersibility in water. In contrast, Comparative Examples 1 and 2 that did not involve the component (b), and Comparative Examples 3 and 4 that did not involve the component (c) resulted in poor dispersibility in water compared to Examples.

Slurries were prepared using a bead mill based on the formulations described in Table 3. Whether the dispersion was successfully prepared was evaluated based on the evaluation criteria below. The results are also described in the table.

### [Success or failure of the preparation of dispersion]

A: The dispersion was successfully obtained.
C: The preparation of dispersion failed.

The sample was judged "Pass" when rated with "A".

### [Evaluation of finish and applicability]

Cosmetics were applied and evaluated by a panel of 10 professional members for finish (not powdery) and applicability (good spreadability) according to the following evaluation criteria. The results of the 10 members were averaged. Each item was rated according to the judgement criteria below.

### [Evaluation criteria]

| | |
|---|---|
| 5 points: | very good |
| 4 points: | good |
| 3 points: | fair |
| 2 points: | slightly poor |
| 1 point: | poor |

The average score was rated according to the following criteria.

### [Judgment criteria]

A: Average score is 4.5 points or more.
B: Average score is 3.5 points or more and less than 4.5 points.
C: Average score is 2.5 points or more and less than 3.5 points.
D: Average score is less than 2.5 points.

The sample was judged "Pass" when rated with "B" or above.

**[Table 3]**

| Formulation (%) | Example | | | | | Comparative Example | | | | |
|---|---|---|---|---|---|---|---|---|---|---|
| | 6 | 7 | 8 | 9 | 10 | 5 | 6 | 7 | 8 | 9 |
| (a) FINEX-33W-LP2 (Note 1) | 60 | 50 | - | - | - | - | 60 | 60 | 60 | 60 |
| (a) MZX-508OTS (Note 2) | - | - | 50 | 50 | 50 | - | - | - | - | - |
| MZ-500HP (Note 3) | - | - | - | - | - | 50 | - | - | - | - |
| (b) BG | 10 | 10 | 10 | - | 20 | 20 | 15 | 10 | 10 | 10 |
| (b) Glycerin | - | - | - | 10 | - | - | - | - | - | - |
| (c) KF-6100 (Note 4) | 5 | 4 | 4 | 4 | 4 | 4 | - | - | - | - |
| KF-6106 (Note 5) | - | - | - | - | - | - | - | 5 | - | - |
| KF-6017 (Note 6) | - | - | - | - | - | - | - | - | 5 | - |
| KF-6013 (Note 7) | - | - | - | - | - | - | - | - | - | 5 |
| (d) Water | 25 | 36 | 36 | 36 | 26 | 26 | 25 | 25 | 25 | 25 |
| Total | 100 | 100 | 100 | 100 | 100 | 100 | 100 | 100 | 100 | 100 |
| (c)/(b) | 0.5 | 0.4 | 0.4 | 0.4 | 0.2 | 0.2 | - | - | - | - |
| Feasibility of dispersion preparation | A | A | A | A | A | C | C | C | C | A |
| Finish | A | A | A | A | A | - | - | - | - | C |
| Applicability | B | B | A | B | B | - | - | - | - | C |

| | |
|---|---|
| (Note 1) | Fine particles of zinc oxide (labeling name, INCI: Zinc Oxide) treated with hydrous silica (labeling name, INCI: Hydrated Silica) and with hydrogen dimethicone (labeling name, INCI: Hydrogen Dimethicone), number average primary particle diameter determined by image analysis of a transmission electron photomicrograph: 35 nm (average diameter of 200 particles measured by image analysis of a transmission electron photomicrograph) |
| (Note 2) | Fine particles of zinc oxide (labeling name, INCI: Zinc Oxide) treated with triethoxycaprylylsilane (labeling name, INCI: Triethoxycaprylylsilane), number average primary particle diameter determined by image analysis of a transmission electron photomicrograph: 25 nm (average diameter of 200 particles measured by image analysis of a transmission electron photomicrograph) |
| (Note 3) | Fine particles of zinc oxide (labeling name, INCI: Zinc Oxide) treated with hydrous silica (labeling name, INCI: Hydrated Silica), number average primary particle diameter determined by image analysis of a transmission electron photomicrograph: 25 nm (average diameter of 200 particles measured by image analysis of a transmission electron photomicrograph) |
| (Note 4) | Polyglyceryl-3 disiloxane dimethicone (labeling name, INCI: Polyglyceryl-3 Disiloxane Dimethicone) |
| (Note 5) | Polyglyceryl-3 polydimethylsiloxyethyl dimethicone (labeling name, INCI: Polyglyceryl-3 Polydimethylsiloxyethyldimethicone), insoluble in components (b) |
| (Note 6) | PEG-10 dimethicone (labeling name, INCI: PEG-10 Dimethicone) |
| (Note 7) | PEG-9 dimethicone (labeling name, INCI: PEG-9 Dimethicone) |

As is clear from Table 3, dispersions (slurries) were successfully obtained in all of Examples 6 to 10 of the present invention. In contrast, the preparation of dispersion failed in Comparative Example 5 that did not involve the component (a), and in Comparative Examples 6 to 8 that did not involve the component (c). Comparative Example 9, in which a water-miscible polyether-modified silicone was added, resulted in a powdery finish and poor applicability.

Dispersions were prepared based on the formulations and the dispersing methods described in Table 4 below. Whether the dispersion was successfully prepared was evaluated in the same manner as above.

**[Table 4]**

| Formulation (%) | Example | | | | | | | |
|---|---|---|---|---|---|---|---|---|
| | 11 | 12 | 13 | 14 | 15 | 16 | 17 | 18 |
| (a) FINEX-52W-LP2 (Note 1) | 50 | 50 | - | - | 20 | 60 | 60 | 80 |
| (a) FINEX-33W-LP2 (Note 2) | - | - | 50 | 50 | - | - | - | - |
| (b) BG | 35 | 45 | 35 | 45 | 5 | 10 | 10 | 15 |
| (c) KF-6100 (Note 3) | 5 | 5 | 5 | 5 | 3 | 5 | 5 | 5 |
| (d) Water | 10 | - | 10 | - | Balance | Balance | Balance | - |
| Simethicone | - | - | - | - | 0.005 | 0.005 | 0.005 | - |
| Preservative | - | - | - | - | q.s. | - | q.s. | - |
| Total | 100 | 100 | 100 | 100 | 100 | 100 | 100 | 100 |
| Disperser | High-pressure disperser | High-pressure disperser | High-pressure disperser | High-pressure disperser | Bead mill | Bead mill | Bead mill | Roll mill |
| Feasibility of dispersion preparation | A | A | A | A | A | A | A | A |

| | |
|---|---|
| (Note 1) | Fine particles of zinc oxide (labeling name, INCI: Zinc Oxide) treated with hydrous silica (labeling name, INCI: Hydrated Silica) and with hydrogen dimethicone (labeling name, INCI: Hydrogen Dimethicone), number average primary particle diameter determined by image analysis of a transmission electron photomicrograph: 20 nm (average diameter of 200 particles measured by image analysis of a transmission electron photomicrograph) |
| (Note 2) | Fine particles of zinc oxide (labeling name, INCI: Zinc Oxide) treated with hydrous silica (labeling name, INCI: Hydrated Silica) and with hydrogen dimethicone (labeling name, INCI: Hydrogen Dimethicone), number average primary particle diameter determined by image analysis of a transmission electron photomicrograph: 35 nm (average diameter of 200 particles measured by image analysis of a transmission electron photomicrograph) |
| (Note 3) | Polyglyceryl-3 disiloxane dimethicone (labeling name, INCI: Polyglyceryl-3 Disiloxane Dimethicone) |

As is clear from Table 4, dispersions were successfully obtained in all of Examples 11 to 18 of the present invention with a high-pressure disperser, a bead mill or a roll mill. The dispersions obtained were rated "A" in the dispersion preparation feasibility test described above. All the dispersions were stable with good water dispersibility, offered an excellent finish, and exhibited excellent applicability.

### [Reference Examples]

Based on the formulations described in Table 5 below, dispersions were prepared in the same manner as in Example 1. The feasibility of the preparation of dispersion was tested in the same manner as above.

**[Table 5]**

| Formulation (%) | Reference Example | | |
|---|---|---|---|
| | 1 | 2 | 3 |
| (e) STR-100W-LP (Note 1) | 50 | 50 | 50 |
| (b) BG | 10 | 10 | 13 |
| (c) KF-6100 (Note 2) | 7 | - | - |
| KF-6013 (Note 3) | - | 7 | 12 |
| (d) Water | 33 | 33 | 25 |
| Total | 100 | 100 | 100 |
| Feasibility of dispersion preparation | A | C | A |

| | |
|---|---|
| (Note 1) | Fine particles of titanium dioxide (labeling name, INCI: Titanium Dioxide) treated with hydrous silica (labeling name, INCI: Hydrated Silica) and with hydrogen dimethicone (labeling name, INCI: Hydrogen Dimethicone), number average primary particle diameter: 15 nm |
| (Note 2) | Polyglyceryl-3 disiloxane dimethicone (labeling name, INCI: Polyglyceryl-3 Disiloxane Dimethicone) |
| (Note 3) | PEG-9 dimethicone (labeling name, INCI: PEG-9 Dimethicone) |

### [Examples 19 to 21 and Comparative Examples 10 and 11]

O/W creams were prepared according to the formulations described in Table 6 below. The O/W creams obtained were evaluated as described later.

**[Table 6]**

| Formulation (%) | | Example | | | Comparative Example | |
|---|---|---|---|---|---|---|
| | | 19 | 20 | 21 | 10 | 11 |
| (1) | Dispersion of Example 5 ((a) 60%) | 10 | 10 | 10 | - | - |
| | Dispersion of Comparative Example 9 ((a) 60%) | - | - | - | 10 | - |
| | Dispersion of Reference Example 1 ((e) 50%) | 10 | 20 | - | - | - |
| | Dispersion of Reference Example 3 ((e) 50%) | - | - | - | 10 | - |
| (2) | Dimethicone | 3 | 3 | 3 | 3 | 3 |
| | SPD-T7 (Note 1) ((e) 45%) | - | - | 22 | - | - |
| | (Ammonium acryloyldimethyl taurate/VP) copolymer | 0.2 | 0.2 | 0.2 | 0.2 | 0.2 |
| | (Sodium acrylate/sodium acryloyldimethyl taurate) copolymer mixture | 2 | 2 | 2 | 2 | 2 |
| | Polyglyceryl-10 myristate | 0.2 | 0.2 | 0.2 | 0.2 | 0.2 |
| | Polyglyceryl-10 diisostearate | 0.8 | 0.8 | 0.8 | 0.8 | 0.8 |
| (3) | (a) FINEX-33W-LP2 (Note 2) | - | - | - | - | 6 |
| | (e) STR-100W-LP (Note 3) | - | - | - | - | 5 |
| | (c) KF-6100 (Note 4) | - | - | - | - | 1.2 |
| | BG | 6 | 6 | 6 | 6 | 8 |
| | Ethanol | 6 | 6 | 6 | 6 | 6 |
| | Phenoxyethanol | 0.2 | 0.2 | 0.2 | 0.2 | 0.2 |
| | Water | 61.6 | 51.6 | 49.6 | 61.6 | 67.4 |
| Total | | 100 | 100 | 100 | 100 | 100 |
| Transparency | | A | A | A | A | D |
| Feeling on use | | A | B | A | D | D |
| Water resistance | | A | A | A | C | A |

| | |
|---|---|
| (Note 1) | 45% Cyclopentasiloxane dispersion of fine particles of titanium dioxide (labeling name, INCI: Titanium dioxide) treated with aluminum hydroxide (labeling name, INCI: Hydrated Aluminum) and with stearic acid (labeling name, INCI: Stearic Acid), number average primary particle diameter: 20 nm, Shin-Etsu Chemical Co., Ltd. |
| (Note 2) | Fine particles of zinc oxide (labeling name, INCI: Zinc Oxide) treated with hydrous silica (labeling name, INCI: Hydrated Silica) and with hydrogen dimethicone (labeling name, INCI: Hydrogen Dimethicone), number average primary particle diameter: 35 nm |
| (Note 3) | Fine particles of titanium dioxide (labeling name, INCI: Titanium Dioxide) treated with hydrous silica (labeling name, INCI: Hydrated Silica) and with hydrogen dimethicone (labeling name, INCI: Hydrogen Dimethicone), number average primary particle diameter: 15 nm |
| (Note 4) | Polyglyceryl-3 disiloxane dimethicone (labeling name, INCI: Polyglyceryl-3 Disiloxane Dimethicone) |

### [Production method]

A: Components (2) were mixed.
B: Components (3) were mixed.
C: The composition obtained in A was added to the composition obtained in B and emulsified.
D: The component(s) (1) was added to the emulsion obtained in C. The resultant mixture was mixed to give an O/W cream.

### [Evaluation of transparency and the feeling on use]

The cosmetics were evaluated by a panel of 10 professional members for transparency upon application and the feeling on use (non-stickiness) according to the following evaluation criteria. The results of the 10 members were averaged. Each item was rated according to the judgement criteria below.

### [Evaluation criteria]

| | |
|---|---|
| 5 points: | very good |
| 4 points: | good |
| 3 points: | fair |
| 2 points: | slightly poor |
| 1 point: | poor |

The average score was rated according to the following criteria.

### [Judgment criteria]

A: Average score is 4.5 points or more.
B: Average score is 3.5 points or more and less than 4.5 points.
C: Average score is 2.5 points or more and less than 3.5 points.
D: Average score is less than 2.5 points.

The sample was judged "Pass" when rated with "B" or above.

### [Evaluation of water resistance]

The water resistance of the cosmetics that were applied was evaluated according to the following evaluation criteria. The results were judged according to the criterion below.

### [Judgement criteria]

A: Remains after exposed to running water for 1 minute.
C: Vanishes after exposed to running water for 1 minute.

The sample was judged "Pass" when rated with "A".

As is clear from the results in Table 6, the cosmetics of Examples 19 to 21 that included the aqueous dispersion of the present invention were excellent in transparency, feeling on use, and water resistance. In contrast, the cosmetic of Comparative Example 10 that used the dispersion of Comparative Example 9 was poor in feeling on use and water resistance. In Comparative Example 11, the components for the cosmetic were the same as those in Example 19 but did not form the aqueous dispersion of the present invention, and the cosmetic thus obtained was inferior in transparency and feeling on use. Furthermore, both the cosmetics of Examples 20 and 21 achieved excellent transparency, feeling on use, and water resistance, and exhibited UV-shielding effects against a wide range from UVA to UVB by virtue of the high loading of (e) fine particles of titanium dioxide. In particular, the cosmetic of Example 21 that contained (e) fine particles of titanium dioxide in the oil phase outperformed the cosmetic of Example 20 in feeling on use and stability.

### [Example 22] Oil-in-water sunscreen

| | Formulation | % |
|---|---|---|
| -. | KSG-15 (Note 1) | 8.0 |
| 2. | KSG-16 (Note 2) | 19.0 |
| 3. | KSG-45 (Note 3) | 5.0 |
| 4. | Cyclopentasiloxane | 5.0 |
| 5. | Ethylhexyl methoxycinnamate | 5.0 |
| 6. | BG | 3.0 |
| 7. | KF-6100 (Note 4) | 0.6 |
| 8. | KF-6104 (Note 5) | 0.3 |
| 9. | Aqueous solution of (ammonium acryloyldimethyl taurate/VP) copolymer (Note6) | 13.0 |
| 10. | Aqueous solution of (acrylamide/sodium acryloyldimethyl taurate) copolymer/isohexadecane/polysorbate 80 (Note 7) | 0.6 |
| 11. | 1% Aqueous solution of sodium chloride | 8.0 |
| 12. | Water | 12.5 |
| 13. | Dispersion of Example 6 | 10.0 |
| 14. | Dispersion of Reference Example 1 | 10.0 |
| | Total | 1 100.0 |

| | |
|---|---|
| (Note 1) | Mixture of 90-96% cyclopentasiloxane + 4-10% (dimethicone/vinyl dimethicone) crosspolymer, Shin-Etsu Chemical Co., Ltd. |
| (Note 2) | Mixture of 70-80% dimethicone + 20-30 wt% (dimethicone/vinyl dimethicone) crosspolymer, Shin-Etsu Chemical Co., Ltd. |
| (Note 3) | Mixture of 60-70 wt% cocoalkyl (caprylate/caprate) + 30-40 wt% (dimethicone/vinyl dimethicone) crosspolymer, Shin-Etsu Chemical Co., Ltd. |
| (Note 4) | Polyglyceryl-3 disiloxane dimethicone, Shin-Etsu Chemical Co., Ltd. |
| (Note 5) | Polyglyceryl-3 polydimethylsiloxyethyl dimethicone, Shin-Etsu Chemical Co., Ltd. |
| (Note 6) | Aristoflex AVC, Clariant |
| (Note 7) | Simulgel 600, Seppic |

### (Production method)

A: Components 1 to 5 were mixed uniformly.
B: Components 6 to 12 were mixed uniformly.
C: The mixture obtained in A was added to the mixture obtained in B and emulsified. Components 13 and 14 were added. The resultant mixture was dispersed uniformly.

The oil-in-water sunscreen obtained as described above was excellent in transparency, feeling on use, and water resistance.

### [Example 23] Oil-in-water base cream

| | Formulation | % |
|---|---|---|
| 1. | Water | Balance |
| 2. | Glycerin | 3.0 |
| 3. | Microcrystalline wax | 3.0 |
| 4. | Xanthan gum | 0.2 |
| 5. | Pentylene glycol | 2.0 |
| 6. | BG | 5.0 |
| 7. | Dispersion of Example 16 | 10.0 |
| 8. | Sucrose cocoate | 0.2 |
| 9. | Sorbitan stearate | 3.0 |
| 10. | PEG-60 glyceryl isostearate | 0.5 |
| 11. | Behenyl alcohol | 0.5 |
| 12. | Ethylhexyl palmitate | 3.0 |
| 13. | KSP-101 (Note 1) | 3.0 |
| 14. | Triethylhexanoin | 6.0 |
| 15. | Polyhydroxystearic acid | 0.5 |
| 16. | KTP-09W (Note 2) | q.s. |
| 17. | KTP-09R (Note 2) | q.s. |
| 18. | KTP-09Y (Note 2) | q.s. |
| 19. | KTP-09B (Note 2) | q.s. |
| 20. | Polysorbate 60 | 0.3 |
| 21. | (Hydroxyethyl acrylate/sodium acryloyldimethyl taurate) copolymer (Note 3) | 0.6 |
| | Total | 100.0 |

| | |
|---|---|
| (Note 1) | (Vinyl dimethicone/methicone silsesquioxane) crosspolymer, Shin-Etsu Chemical Co., Ltd. |
| (Note 2) | KF-9909-treated coloring inorganic pigments, W: white, R: red, Y: yellow, B: black, Shin-Etsu Chemical Co., Ltd. |
| (Note 3) | SIMULGEL EG, SEPPIC |

### (Production method)

A: Components 1 to 6 were mixed uniformly, and component 7 was added. The resultant mixture was mixed uniformly.
B: Components 8 to 12 were heated to give a solution, and component 13 was added. The resultant mixture was mixed uniformly.
C: Components 14 to 19 were mixed and roll-treated.
D: The mixture obtained in step C was added to the mixture obtained in step B. The resultant mixture was mixed uniformly.
E: The hot mixture obtained in step D was added to the hot mixture obtained in step A. The resultant mixture was emulsified uniformly.
F: The emulsion obtained in step E was cooled to room temperature, and components 20 and 21 were added. The resultant mixture was mixed uniformly.

The oil-in-water base cream obtained as described above was highly stable, had a non-sticky comfortable feeling on use, and was excellent in water resistance.

### [Example 24] Aqueous gel

| | Formulation | % |
|---|---|---|
| 1. | Dispersion of Example 4 | 5.0 |
| 2. | Ethanol | 3.5 |
| 3. | BG | 4.0 |
| 4. | Glycerin | 2.0 |
| 5. | (Ammonium acryloyldimethyl taurate/VP) copolymer | 0.2 |
| 6. | Xanthan gum | 0.2 |
| 7. | KSP-100W (Note 1) | 1.0 |
| 8. | Phenoxyethanol | 0.3 |
| 9. | Water | Balance |
| | Total | 100.0 |

| | | |
|---|---|---|
| (Note 1) Hydrophilized powder of (vinyl dimethicone/methicone silsesquioxane) crosspolymer, Shin-Etsu Chemical Co., Ltd. | | |

### (Production method)

A: Components 1 and 2 were mixed uniformly.
B: Components 3 to 9 were mixed uniformly.
C: The mixture obtained in step A was added to the mixture obtained in step B. The resultant mixture was mixed uniformly.
D: The mixture obtained in step C was degassed and was then added into a container. An aqueous gel was thus obtained.

The aqueous gel obtained as described above was excellent in transparency, feeling on use, and water resistance. Component 1 was highly stable and was easy to handle and admit.

### [Example 25] Oil-in-water sunscreen milky lotion

| Formulation | | % |
|---|---|---|
| 1. | Dimethicone-treated fine particles of zinc oxide | 7.5 |
| 2. | 1,2-Hexanediol | 0.5 |
| 3. | BG | 4.0 |
| 4. | KF-6100 (Note 1) | 1.2 |
| 5. | KM-116 (Note 2) | 0.5 |
| 6. | Sorbitol | 3.0 |
| 7. | Ethanol | 9.5 |
| 8. | BG | 5.0 |
| 9. | Sodium acrylate/sodium acryloyldimethyl taurate copolymer composition (Note 3) | 2.5 |
| 10. | KM-440 (Note 4) | 2.0 |
| 11. | Water | Balance |
| 12. | KF-7312J (Note 5) | 1.0 |
| 13. | KF-56A (Note 6) | 3.0 |
| 14. | Cetanol | 2.0 |
| 15. | Homosalate | 5.0 |
| 16. | Diethylamino hydroxybenzoyl hexyl benzoate | 1.0 |
| 17. | Polyoxyethylene (60) hydrogenated castor oil | 1.0 |
| 18. | KF-6011 (Note 7) | 0.5 |
| 19. | Tocopherol | 0.05 |
| Total | | 100.0 |

| | |
|---|---|
| (Note 1) | Polyglyceryl-3 disiloxane dimethicone, Shin-Etsu Chemical Co., Ltd. |
| (Note 2) | Dispersion of (dimethicone/vinyl dimethicone) crosspolymer in water, Shin-Etsu Chemical Co., Ltd. |
| (Note 3) | SIMULGEL EG, SEPPIC |
| (Note 4) | Dispersion of (vinyl dimethicone/lauryl dimethicone) crosspolymer in water, Shin-Etsu Chemical Co., Ltd. |
| (Note 5) | 50% Cyclopentasiloxane solution of trimethylsiloxysilicate, Shin-Etsu Chemical Co., Ltd. |
| (Note 6) | Diphenylsiloxyphenyl trimethicone, Shin-Etsu Chemical Co., Ltd. |
| (Note 7) | PEG-11 methyl ether dimethicone, Shin-Etsu Chemical Co., Ltd. |

### (Production method)

A: Components 1 to 4 were dispersed uniformly using a roll mill.
B: Components 5 to 11 were heated to 85°C, and the mixture obtained in A was added. The resultant mixture was mixed uniformly.
C: Components 12 to 19 were heated to 85°C and mixed uniformly.
D: The mixture obtained in C was added to the mixture obtained in B and emulsified at 85°C. While performing stirring, the emulsion was cooled slowly. An oil-in-water sunscreen milky lotion was thus obtained.

The aqueous gel of oil-in-water sunscreen milky lotion obtained as described above was excellent in transparency, feeling on use, and water resistance. The dispersion of the present invention prepared in A in the production method was highly stable and was easy to handle and admit.

### [Example 26] W/O base

| Formulation | | % |
|---|---|---|
| 1. | KSG-270 (Note 1) | 3.0 |
| 2. | KSG-18A (Note 2) | 3.0 |
| 3. | KF-6048 (Note 3) | 2.0 |
| 4. | KF-56A (Note 4) | 5.0 |
| 5. | KF-4422 (Note 5) | Balance |
| 6. | Ethylhexyl methoxycinnamate | 5.0 |
| 7. | Ethylhexyl salicylate | 2.0 |
| 8. | Octocrylene | 1.0 |
| 9. | Diethylamino hydroxybenzoyl hexyl benzoate | 2.0 |
| 10. | KSP-105 (Note 6) | 2.0 |
| 11. | SPD-T7 (Note 7) | 10.0 |
| 12. | KF-6100 (Note 8) | 0.4 |
| 13. | BG | 2.0 |
| 14. | Silicone-treated fine particles of zinc oxide | 9.0 |
| 15. | Water | 10.0 |
| 16. | Sodium chloride | 0.3 |
| 17. | Sodium citrate | 0.1 |
| 18. | Ethanol | 3.0 |
| Total | | 100.0 |

| | |
|---|---|
| (Note 1) | Mixture of diphenylsiloxyphenyl trimethicone + 15-25% (dimethicone/(PEG-10/15)) crosspolymer, Shin-Etsu Chemical Co., Ltd. |
| (Note 2) | Mixture of diphenylsiloxyphenyl trimethicone + 10-20% (dimethicone/phenylvinyl dimethicone) crosspolymer, Shin-Etsu Chemical Co., Ltd. |
| (Note 3) | Cetyl PEG/PPG-10/1 dimethicone, Shin-Etsu Chemical Co., Ltd. |
| (Note 4) | Diphenylsiloxyphenyl trimethicone, Shin-Etsu Chemical Co., Ltd. |
| (Note 5) | Ethyl trimethicone, Shin-Etsu Chemical Co., Ltd. |
| (Note 6) | (Vinyl dimethicone/methicone silsesquioxane) crosspolymer, Shin-Etsu Chemical Co., Ltd. |
| (Note 7) | 45% Cyclopentasiloxane dispersion of fine particles of titanium dioxide, Shin-Etsu Chemical Co., Ltd. |
| (Note 8) | Polyglyceryl-3 disiloxane dimethicone, Shin-Etsu Chemical Co., Ltd. |

### (Production method)

A: Components 1 to 10 were mixed uniformly.
B: Components 15 to 17 were mixed, and a mixture of components 12 and 13 prepared beforehand was added. Component 14 was added. The resultant mixture was dispersed using a homomixer.
C: The mixture obtained in B and component 18 were mixed uniformly.
D: The mixture obtained in C was added to the mixture obtained in A and emulsified.
E: Component 11 was added to the mixture obtained in D. The resultant mixture was mixed uniformly.

The W/O base obtained was highly stable, had a non-sticky comfortable feeling on use, and was excellent in water resistance.

### [Example 27] Oil-in-water base

| Formulation | | % |
|---|---|---|
| 1. | KP-545 (Note 1) | 3 |
| 2. | KSG-19 (Note 2) | 5 |
| 3. | KF-56A (Note 3) | 5 |
| 4. | Cyclopentasiloxane | 10 |
| 5. | Dimethicone-treated fine particles of zinc oxide | 7.2 |
| 6. | BG | 1.8 |
| 7. | KF-6100 (Note 4) | 1.6 |
| 8. | Water | 20 |
| 9. | BG | 10 |
| 10. | Betaine | 1 |
| 11. | KF-6043 (Note 5) | 1.5 |
| 12. | Sodium acrylate-sodium acryloyldimethyl taurate copolymer composition (Note 6) | 1 |
| 13. | (Acrylates/alkyl (C10-30) acrylate) crosspolymer (2% aqueous solution) | 20 |
| 14. | Arginine (10% aqueous solution) | q.s. |
| 15. | Bisabolol | 0.1 |
| 16. | Ethylhexylglycerin | 0.1 |
| 17. | EDTA-2Na (10% aqueous solution) | 0.1 |
| 18. | Water | Balance |
| Total | | 100.0 |

| | |
|---|---|
| (Note 1) | 30 wt% cyclopentasiloxane solution of (acrylates/dimethicone) copolymer, Shin-Etsu Chemical Co., Ltd. |
| (Note 2) | Mixture of 80-90 wt% dimethicone + 10-20 wt% (dimethicone/vinyl dimethicone) crosspolymer, Shin-Etsu Chemical Co., Ltd. |
| (Note 3) | Diphenylsiloxyphenyl trimethicone, Shin-Etsu Chemical Co., Ltd. |
| (Note 4) | Polyglyceryl-3 disiloxane dimethicone, Shin-Etsu Chemical Co., Ltd. |
| (Note 5) | PEG-10 dimethicone, Shin-Etsu Chemical Co., Ltd. |
| (Note 6) | SIMULGEL EG, SEPPIC |

### (Production method)

A: Components 1 to 4 were mixed uniformly.
B: Components 5 to 8 were dispersed uniformly using a homomixer.
C: Components 9 to 18 and the dispersion obtained in B were mixed uniformly.
D: The mixture obtained in A was added to the mixture obtained in C and emulsified to give an O/W base.

The oil-in-water base obtained as described above was highly stable, had a non-sticky comfortable feeling on use, and was excellent in water resistance. The dispersion obtained in B was highly stable and was easy to handle and admit.

### [Example 28] Oil-in-water liquid foundation

| Formulation | | % |
|---|---|---|
| 1. | Stearic acid | 1.0 |
| 2. | Behenyl alcohol | 0.4 |
| 3. | Glyceryl stearate | 0.3 |
| 4. | Mineral oil | 10.0 |
| 5. | Glyceryl trioctanoate | 5.0 |
| 6. | KP-561P (Note 1) | 3.0 |
| 7. | Sorbitan sesquioleate | 0.5 |
| 8. | Sorbitan monooleate | 1.0 |
| 9. | Acrylates copolymer | 2.2 |
| 10. | Triethanolamine | 1.0 |
| 11. | KF-6013 (Note 2) | 0.2 |
| 12. | Alkyl·POE palmityl ether phosphate | 0.1 |
| 13. | POE hydrogenated castor oil | 0.5 |
| 14. | Silicone-treated titanium dioxide (Note 3) | 8.5 |
| 15. | Silicone-treated red iron oxide (Note 3) | 0.4 |
| 16. | Silicone-treated yellow iron oxide (Note 3) | 1.0 |
| 17. | Silicone-treated black iron oxide (Note 3) | 0.1 |
| 18. | Dimethicone-treated fine particles of zinc oxide | 5.0 |
| 19. | DPG | 3.0 |
| 20. | KF-6100 (Note 4) | 0.7 |
| 21. | Water | 3.3 |
| 22. | BG | 7.0 |
| 23. | Preservative | q.s. |
| 24. | Fragrance | q.s. |
| 25. | Water | Balance |
| Total | | 100.0 |

| | |
|---|---|
| (Note 1) | (Acrylates/stearyl acrylate/dimethicone methacrylate) copolymer, Shin-Etsu Chemical Co., Ltd. |
| (Note 2) | PEG-9 dimethicone, Shin-Etsu Chemical Co., Ltd. |
| (Note 3) | Treated with triethoxycaprylylsilane, Shin-Etsu Chemical Co., Ltd. |
| (Note 4) | Polyglyceryl-3 disiloxane dimethicone, Shin-Etsu Chemical Co., Ltd. |

### (Production method)

A: Components 11 to 13 were mixed with a portion of component 22, and components 14 to 17 were added. The resultant mixture was dispersed uniformly and heated.
B: Components 1 to 8 were mixed and heated to give a uniform solution.
C: Components 9 and 10, the remainder of component 22, and components 23 and 25 were mixed and heated.
D: Components 18 to 21 were dispersed using a high-pressure disperser.
E: While performing stirring, the solution obtained in B was added to the mixture obtained in C and emulsified. The dispersion obtained in A was added, and further the dispersion obtained in D and component 24 were added. An oil-in-water liquid foundation was thus obtained.

The oil-in-water liquid foundation obtained as described above was highly stable, had a non-sticky comfortable feeling on use, and was excellent in water resistance. The dispersion obtained in D was highly stable and was easy to handle and admit.

### [Example 29] Water-in-oil sunscreen milky lotion

| Formulation | | % |
|---|---|---|
| 1. | KSG-210 (Note 1) | 2.0 |
| 2. | KSG-790 (Note 2) | 1.0 |
| 3. | KSG-15 (Note 3) | 2.0 |
| 4. | KF-6028 (Note 4) | 1.0 |
| 5. | Dimethicone 6 CS | 3.0 |
| 6. | KF-4418 (Note 5) | 2.0 |
| 7. | Cyclopentasiloxane | 5.0 |
| 8. | Isotridecyl isononanoate | 4.0 |
| 9. | SPD-T5 (Note 6) | 5.0 |
| 10. | SPD-Z7L (Note 7) | 20.0 |
| 11. | Sodium citrate | 0.2 |
| 12. | Sodium chloride | 0.5 |
| 13. | Dispersion of Example 9 | 20.0 |
| 14. | Preservative | q.s. |
| 15. | Fragrance | q.s. |
| 16. | Water | Balance |
| Total | | 100.0 |

| | |
|---|---|
| (Note 1) | Mixture of 70-80 wt% dimethicone + 20-30 wt% (dimethicone/(PEG-10/15)) crosspolymer, Shin-Etsu Chemical Co., Ltd. |
| (Note 2) | Mixture of 65-75 wt% caprylyl methicone + 25-35 wt% (dimethicone/polyglycerin-3) crosspolymer, Shin-Etsu Chemical Co., Ltd. |
| (Note 3) | Mixture of 90-96 wt% cyclopentasiloxane + 4-10 wt% (dimethicone/vinyl dimethicone) crosspolymer, Shin-Etsu Chemical Co., Ltd. |
| (Note 4) | PEG-9 polydimethylsiloxyethyl dimethicone, Shin-Etsu Chemical Co., Ltd. |
| (Note 5) | Caprylyl methicone, Shin-Etsu Chemical Co., Ltd. |
| (Note 6) | 40 wt% cyclopentasiloxane dispersion of fine particles of titanium dioxide, Shin-Etsu Chemical Co., Ltd. |
| (Note 7) | 60 wt% dimethicone dispersion of fine particles of zinc oxide, Shin-Etsu Chemical Co., Ltd. |

### (Production method)

A: Components 1 to 8 were mixed uniformly.
B: Components 11 to 16 were mixed uniformly.
C: While performing stirring, the mixture obtained in A was added to the mixture obtained in B and emulsified. Components 9 and 10 were added. A water-in-oil sunscreen milky lotion was thus obtained.

The water-in-oil sunscreen milky lotion obtained as described above was highly stable, had a highly transparent, non-sticky comfortable feeling on use, and was excellent in water resistance. Component 13 was highly stable and was easy to handle and admit.

### [Example 30] Oil-in-water sunscreen milky lotion

| | Formulation | % |
|---|---|---|
| 1. | Hydrous silica·hydrogen dimethicone-treated fine particles of zinc oxide | 10 |
| 2. | BG | 2 |
| 3. | KF-6100 (Note 1) | 1.4 |
| 4. | KM-72 (Note 2) | 0.001 |
| 5. | Water | 6.599 |
| 6. | Cyclopentasiloxane | 10 |
| 7. | KF-56A (Note 3) | 3 |
| 8. | Cetanol | 0.5 |
| 9. | Polyoxyethylene sorbitan monooleate | 2.5 |
| 10. | Glyceryl stearate (SE) | 0.5 |
| 11. | KF-6011 (Note 4) | 1 |
| 12. | DPG | 6 |
| 13. | BG | 6 |
| 14. | Carboxyvinyl polymer | 0.3 |
| 15. | Acrylic polymer compound | 0.3 |
| 16. | Methylparaben | 0.2 |
| 17. | Phenoxyethanol | 0.3 |
| 18. | EDTA-2Na | q.s. |
| 19. | Water | Balance |
| 20. | Sodium hydroxide (10% aqueous solution) | q.s. |
| 21. | KM-116 (Note 5) | 2 |
| | Total | 100.0 |

| | |
|---|---|
| (Note 1) | Polyglyceryl-3 disiloxane dimethicone, Shin-Etsu Chemical Co., Ltd. |
| (Note 2) | Simethicone emulsion, Shin-Etsu Chemical Co., Ltd. |
| (Note 3) | Diphenylsiloxyphenyl trimethicone, Shin-Etsu Chemical Co., Ltd. |
| (Note 4) | PEG-11 methyl ether dimethicone, Shin-Etsu Chemical Co., Ltd. |
| (Note 5) | (Dimethicone/vinyl dimethicone) crosspolymer emulsion, Shin-Etsu Chemical Co., Ltd. |

### (Production method)

A: Components 1 to 5 were dispersed uniformly using a high-pressure disperser.
B: Components 6 to 8 were mixed uniformly.
C: Components 9 to 19 and the dispersion obtained in A were mixed uniformly.
D: The mixture obtained in B was added to the mixture obtained in C and emulsified. Component 20 was added. The resultant mixture was mixed uniformly.
E: Component 21 was added to the mixture obtained in D. The resultant mixture was mixed uniformly.

The oil-in-water sunscreen milky lotion obtained as described above was highly stable, had a highly transparent, non-sticky comfortable feeling on use, and was excellent in water resistance. The dispersion obtained in A was highly stable and was easy to handle and admit.

### [Example 31] Oil-in-water sunscreen milky lotion

| | Formulation | % |
|---|---|---|
| 1. | KTP-09W (Note 1) | 5.0 |
| 2. | Silicone-treated fine particles of zinc oxide (Note 2) | 0.5 |
| 3. | BG | 4.0 |
| 4. | KF-6100 (Note 3) | 1.2 |
| 5. | Ethanol | 0.5 |
| 6. | KM-116 (Note 4) | 0.5 |
| 7. | Ethylhexylglycerin | 0.1 |
| 8. | Ethanol | 9.5 |
| 9. | BG | 5.0 |
| 10. | Sodium acrylate·sodium acryloyldimethyl taurate copolymer composition (Note 5) | 2.5 |
| 11. | KSP-100W (Note 6) | 2.0 |
| 12. | Water | Balance |
| 13. | KF-7312J (Note 7) | 1.0 |
| 14. | KF-56A (Note 8) | 3.0 |
| 15. | Cetanol | 2.0 |
| 16. | Ethylhexyl methoxycinnamate | 5.0 |
| 17. | Diethylamino hydroxybenzoyl hexyl benzoate | 1.0 |
| 18. | Polyoxyethylene (60) hydrogenated castor oil | 1.0 |
| 19. | KF-6011 (Note 9) | 0.5 |
| 20. | Tocopherol | 0.05 |
| | Total | 100.0 |

| | |
|---|---|
| (Note 1) | KF-9909-treated titanium dioxide, Shin-Etsu Chemical Co., Ltd. |
| (Note 2) | AES-3083 (triethoxycaprylylsilane)-treated fine particles of zinc oxide (number average primary particle diameter: 50 nm), Shin-Etsu Chemical Co., Ltd. |
| (Note 3) | Polyglyceryl-3 disiloxane dimethicone, Shin-Etsu Chemical Co., Ltd. |
| (Note 4) | Dispersion of (dimethicone/vinyl dimethicone) crosspolymer in water, Shin-Etsu Chemical Co., Ltd. |
| (Note 5) | SIMULGEL EG, SEPPIC |
| (Note 6) | Hydrophilized powder of (vinyl dimethicone/methicone silsesquioxane) crosspolymer, Shin-Etsu Chemical Co., Ltd. |
| (Note 7) | 50% Cyclopentasiloxane solution of trimethylsiloxysilicate, Shin-Etsu Chemical Co., Ltd. |
| (Note 8) | Diphenylsiloxyphenyl trimethicone, Shin-Etsu Chemical Co., Ltd. |
| (Note 9) | PEG-11 methyl ether dimethicone, Shin-Etsu Chemical Co., Ltd. |

### (Production method)

A: Components 1 to 5 were dispersed uniformly using a roll mill.
B: Components 6 to 12 were heated to 85°C, and the mixture obtained in A was added. The resultant mixture was mixed uniformly.
C: Components 13 to 20 were heated to 85°C and mixed uniformly.
D: The mixture obtained in C was added to the mixture obtained in B and emulsified at 85°C. While performing stirring, the emulsion was cooled slowly. An oil-in-water sunscreen milky lotion was thus obtained.

The oil-in-water sunscreen milky lotion obtained as described above was highly stable, had a non-sticky comfortable feeling on use, and was excellent in water resistance. The dispersion of the present invention prepared in A in the production method was excellent in dispersibility, particularly in the aqueous media, water resistance, and feeling on use, and was easy to handle and admit.
The cosmetic was highly stable, had a highly transparent, non-sticky comfortable feeling on use, and was excellent in water resistance. The dispersion obtained in A was highly stable and was easy to handle and admit.

### [Example 32] Oil-in-water sunscreen milky lotion

| | Formulation | % |
|---|---|---|
| 1. | Dimethicone-treated fine particles of zinc oxide | 10 |
| 2. | Pentylene glycol | 2 |
| 3. | KF-6100 (Note 1) | 1.4 |
| 4. | KM-72 (Note 2) | 0.001 |
| 5. | Water | 6.599 |
| 6. | Homosalate | 5 |
| 7. | KF-56A (Note 3) | 3 |
| 8. | Bis-ethylhexyloxyphenol methoxyphenyl triazine | 1.2 |
| 9. | Sorbitan sesquiisostearate | 1.5 |
| 10. | Glyceryl stearate (SE) | 0.5 |
| 11. | Behenyl alcohol | 1 |
| 12. | Polyglyceryl-10 myristate | 3 |
| 13. | BG | 6 |
| 14. | Carboxyvinyl polymer | 0.3 |
| 15. | Acrylic polymer compound | 0.3 |
| 16. | Ethylhexylglycerin | 0.5 |
| 17. | Etidronic acid | q.s. |
| 18. | Hydrophilized fine particles of titanium dioxide | 5 |
| 19. | Water | Balance |
| 20. | Potassium hydroxide (10% aqueous solution) | q.s. |
| | Total | 100.0 |

| | |
|---|---|
| (Note 1) | Polyglyceryl-3 disiloxane dimethicone, Shin-Etsu Chemical Co., Ltd. |
| (Note 2) | Simethicone emulsion, Shin-Etsu Chemical Co., Ltd. |
| (Note 3) | Diphenylsiloxyphenyl trimethicone, Shin-Etsu Chemical Co., Ltd. |

### (Production method)

A: Components 1 to 5 were dispersed uniformly using a bead mill.
B: Components 6 to 11 were mixed uniformly.
C: Component 18 was dispersed in component 19, and then components 12 to 17 and the dispersion obtained in A were mixed uniformly.
D: The mixture obtained in B was added to the mixture obtained in C and emulsified. Component 20 was added. The resultant mixture was mixed uniformly.

The oil-in-water sunscreen milky lotion obtained as described above was highly stable, had a highly transparent, non-sticky comfortable feeling on use, and was excellent in water resistance. The dispersion obtained in A was highly stable and was easy to handle and admit.

## Claims

1. An aqueous dispersion comprising:
(a) 10 to 80% by mass of hydrophobized fine particles of zinc oxide including zinc oxide particles hydrophobized with a silicone, the hydrophobized fine particles of zinc oxide having a number average primary particle diameter of 8 to 200 nm as determined by image analysis of a transmission electron photomicrograph;
(b) 1 to 50% by mass of an aqueous component having at least two alcoholic hydroxyl groups; and
(c) 1 to 20% by mass of a polyglycerin-modified silicone that dissolves in the component (b).

2. The aqueous dispersion according to claim 1, wherein the total content of the components (a), (b), and (c) in the aqueous dispersion is 90% by mass or more.

3. The aqueous dispersion according to claim 1, wherein the component (a) is hydrophobized fine particles of zinc oxide obtained by treating hydrous silica-coated zinc oxide particles with a silicone.

4. The aqueous dispersion according to claim 1, wherein the silicone in the component (a) is triethoxycaprylylsilane.

5. The aqueous dispersion according to claim 1, wherein the component (b) is an aqueous component having two alcoholic hydroxyl groups.

6. The aqueous dispersion according to claim 1, wherein the component (c) is a component insoluble in water.

7. The aqueous dispersion according to claim 1, wherein the component (c) is polyglyceryl-3 disiloxane dimethicone.

8. The aqueous dispersion according to claim 1, wherein the mass ratio (c)/(b) of the content of the component (c) to the content of the component (b) is 0.2 to 0.9.

9. The aqueous dispersion according to claim 1, wherein the aqueous dispersion further comprises (d) 8 to 82% by mass of water, and the total content of the components (a), (b), (c), and (d) in the aqueous dispersion is 90% by mass or more.

10. A cosmetic comprising the aqueous dispersion described in any one of claims 1 to 9.

11. The cosmetic according to claim 10, wherein the cosmetic is an emulsion comprising an aqueous phase including the aqueous dispersion, and the aqueous phase does not contain (e) hydrophobized fine particles of titanium dioxide that have been hydrophobized and have a number average primary particle diameter of 8 to 200 nm as determined by image analysis of a transmission electron photomicrograph.

12. The cosmetic according to claim 10, wherein the cosmetic is an emulsion comprising an aqueous phase including the aqueous dispersion, and an oil phase containing (e) hydrophobized fine particles of titanium dioxide that have been hydrophobized and have a number average primary particle diameter of 8 to 200 nm as determined by image analysis of a transmission electron photomicrograph.

13. The cosmetic according to claim 10, wherein the cosmetic is an emulsion comprising an aqueous phase including the aqueous dispersion, and the aqueous phase includes a dispersion of hydrophobized fine particles of titanium dioxide that includes:
(e) hydrophobized fine particles of titanium dioxide that have been hydrophobized and have a number average primary particle diameter of 8 to 200 nm as determined by image analysis of a transmission electron photomicrograph;
(b) an aqueous component having at least two alcoholic hydroxyl groups; and
(c) a polyglycerin-modified silicone that dissolves in the component (b).
